# FASCICULE DE BREVET EUROPEEN

(11) **EP 0 644 188 B1**
(45) Date de publication et mention de la délivrance du brevet: **04.12.2002**
(21) Numéro de dépôt: 94400007.4
(22) Date de dépôt: 04.01.1994
(51) Int. Cl.: C07D 233/90, C07F 5/02, C07D 403/10, C07C 311/16, C07C 311/58, C07C 311/29, A61K 31/415

(54) **Nouveau procédé de préparation de dérivés soufrés de l'imidazole et les nouveaux intermédiaires obtenus**
Verfahren zur Herstellung von Schwefelhaltigen Imidazolderivaten und erhaltene Zwischenprodukte
Process for the preparation of sulfur derivatives of imidazole and intermediates obtained

(30) Priorité: 16.09.1993 FR 9311030
(43) Date de publication de la demande: 22.03.1995
(73) Titulaire: Aventis Pharma S.A., 92160 Antony (FR)
(72) Inventeur: Bhatnagar, Neerja, F-91600 Savigny sur Orge (FR); Buendia, Jean, F-94170 Le Perreux sur Marne (FR); Griffoul, Christine, F-93110 Rosny sous Bois (FR)

(56) Documents cités:
- EP-A- 0 044 807
- EP-A- 0 058 476
- EP-A- 0 096 002
- EP-A- 0 112 803
- EP-A- 0 495 627
- JOURNAL OF ORGANIC CHEMISTRY. vol. 23 , 1958 , EASTON US pages 927 - 929 F. J. MARSHALL ET AL 'Some N-arylsulfiônyl-N'-alkylureas'
- TETRAHEDRON vol. 48, no. 47 , 1992 , OXFORD GB pages 10233 - 10238

## Description

La présente invention concerne un nouveau procédé de préparation de dérivés soufrés de l'imidazole et les nouveaux intermédiaires obtenus.

Le document EP 0495627 décrit des dérivés boroniques ayant une activité d'inhibiteurs de l'angiotensine II.

La présente invention a pour objet un nouveau procédé de préparation de produits de formule (I) : 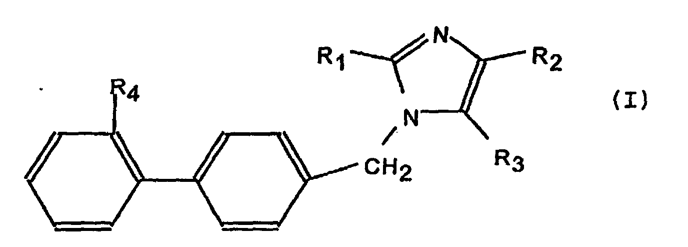 dans laquelle :
R₁ représente un radical alkyle renfermant au plus 4 atomes de carbone,
R₃ représente un atome d'hydrogène, un radical formyle, acyloxy, alkyle ou alcoxy éventuellement substitué, ou un radical carboxy libre, salifié ou estérifié par un radical alkyle,
R₂ représente un radical phénylthio, phénylsulfonyle, phénylsulfinyle, alkylthio, alkylsulfonyle ou alkylsulfinyle, éventuellement substitué, tel que dans tous ces radicaux que peuvent représenter R₂ et R₃, les radicaux alkyle, alcoxy renfermant au plus 6 atomes de carbone, et les radicaux phényle sont éventuellement substitués par un ou plusieurs radicaux choisis parmi les atomes d'halogène et les radicaux hydroxyle, trifluorométhyle, acyloxy, carboxy libre salifié ou estérifié par un radical alkyle, phényle, pyridyle, tétrazolyle, alkyle et alcoxy renfermant au plus 4 atomes de carbone et eux-mêmes éventuellement substitués par un radical alcoxy renfermant au plus 4 atomes de carbone,
R₄ représente le radical cyano, carboxy libre, salifié ou estérifié par un radical alkyle, le radical -SO₂-X-R₁₀ dans lequel X représente les radicaux -NH-, -NH-CO-, -NH-CO-O-, -N=CH-N-R13
-NH-CO-NH- ou une simple liaison et R₁₀ et R₁₃ identiques ou différents, représentent un atome d'hydrogène, un radical méthyle, éthyle, propyle, vinyle, allyle, pyridyle, phényle, benzyle, nitropyridyle, pyrimidyle, tétrazolyle, diazolyle, pipéridinyle, alkylpipéridinyle, thiazolyle, alkylthiazolyle, tétrahydrofuranyle, méthyltétrahydrofuranyle,
lesdits produits de formule (I) étant sous toutes les formes isomères ra cémiques, énantiomères et diastéréoisomères possibles, ainsi que les sels d'addition avec les acides minéraux et organiques ou avec les bases minérales et organiques desdits produits de formule (I), caractérisé en ce que l'on fait réagir un composé de formule (II) : 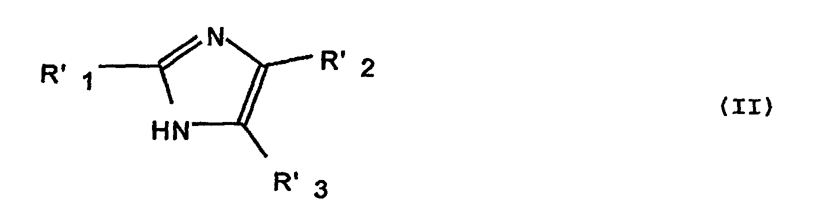 dans laquelle R'₁, R'₂ et R'₃ ont les significations indiquées ci-dessus respectivement pour R₁, R₂ et R₃ et dans lesquelles les éventuelles fonctions réactives sont éventuellement protégées avec un composé de formule (III) : 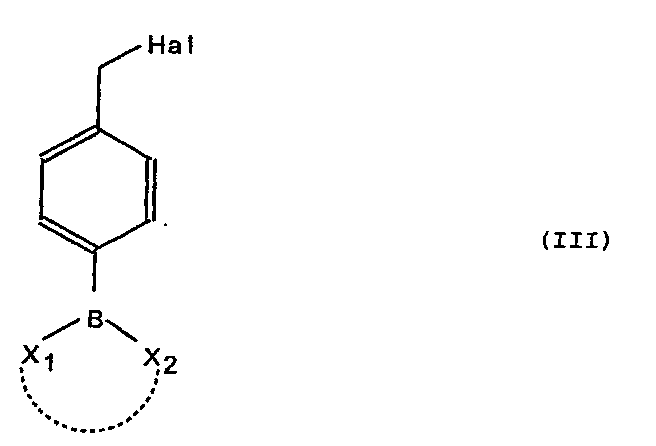 dans laquelle Hal représente un atome d'halogène, B représente un atome de bore et X₁, X₂ sont tels que :
ou bien X₁ et X₂ identiques ou différents représentent un radical hydroxyle, alkyle ou alcoxy renfermant au plus 6 atomes de carbone, un radical phényle ou phénoxy,ou bien X₁ avec X₂ forment avec l'atome de bore auquel ils sont liés un cycle choisi parmi

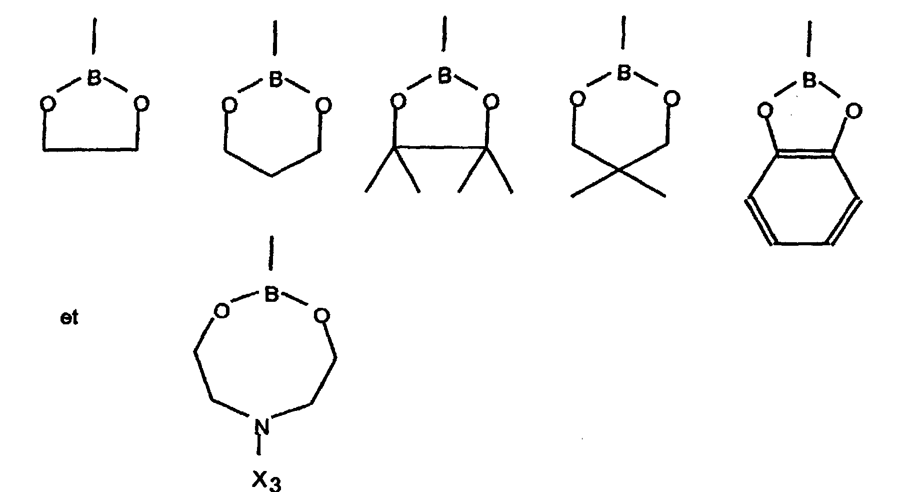

X₃ représentant un atome d'hydrogène ou un radical alkyle renfermant au plus 4 atomes de carbone, pour obtenir un produit de formule (IV) : 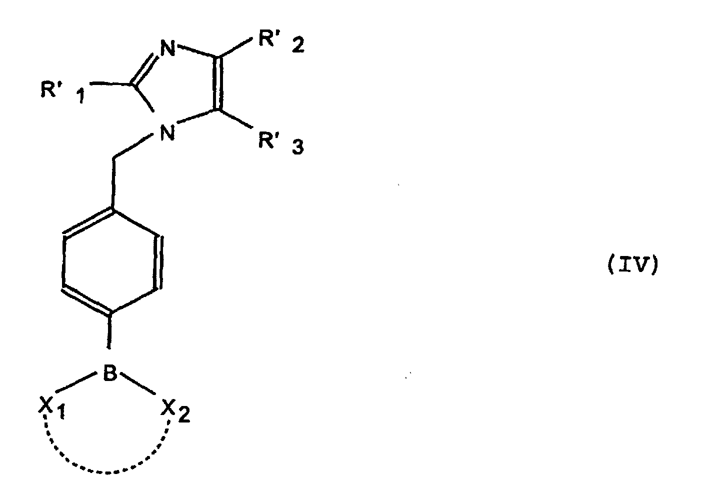 dans laquelle R'₁, R'₂, R'₃, X₁, X₂ et B ont les significations indiquées ci-dessus, produit de formule (IV) que l'on fait réagir avec un produit de formule (V) : 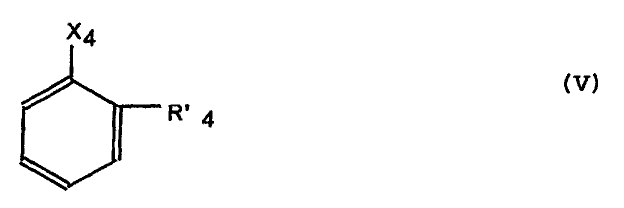 dans lequel X₄ représente un atome d'halogène, un radical alcoxy, triflate ou -O-SO₂F et R'₄ a la signification indiquée ci-dessus pour R₄ dans laquelle les éventuelles fonctions réactives sont éventuellement protégées pour obtenir un produit de formule (I') : 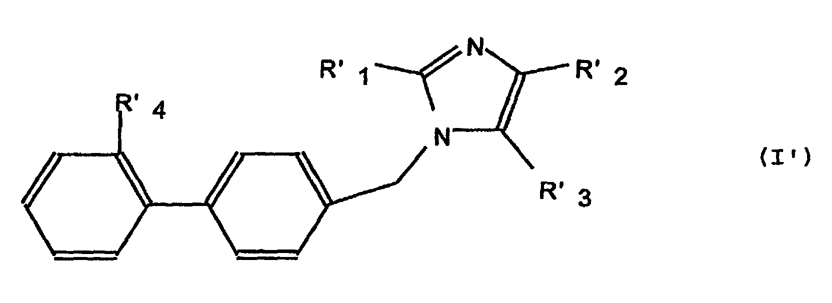 produit de formule (I') que l'on soumet, si désiré et si nécessaire, à l'une ou plusieurs des réactions suivantes, dans un ordre quelconque :
a) une réaction d'élimination des groupements protecteurs que peuvent porter les fonctions réactives protégées,
b) une réaction de salification par un acide minéral ou organique ou par une base,
c) une réaction d'estérification de fonction acide,
d) une réaction de saponification de fonction ester,
e) une réaction de transformation de la fonction cyano en fonction acide,
f) une réaction de réduction de la fonction carboxy en fonction alcool,
g) une réaction de transformation de fonction alcoxy en fonction hydroxyle,
h) une réaction d'oxydation de groupement comprenant un atome de soufre en fonction sulfoxyde ou sulfone correspondante,
i) une réaction de transformation de fonction sulfoxyde ou sulfone en fonction sulfoximine correspondante,
j) une réaction de transformation de radical nitrile en tétrazole,
k) une réaction de dédoublement des formes racémiques en produits dédoublés,
l) une réaction de transformation du radical carboxy en radical carbamoyle,
m) une réaction de transformation du radical carbamoyle en radical nitrile,
lesdits produits de formule (I) ainsi obtenus étant sous toutes les formes isomères possibles racémiques, énantiomères et diastéréoisomères.

Parmi les valeurs de -S(O)ₘ₂-X-R₁₀, on peut citer par exemple et de façon non exhaustive les radicaux : -SO₂-NH₂, -SO₂-NH-CH₃, -SO₂-NH-CF₃, -SO₂-NH-C₆H₅, -SO₂-NH-CH₂-C₆H₅,-CH₂-SO₂-NH₂, -CH₂-SO₂-NH-C₆H₅, -SO₂-NH-CO-NH-CH₃, -SO₂-NH-CO-NH-C₆H₅, -SO₂-MH-CO-NH-CF₃, -SO₂-NH-CO-NH-CH₂-C₆H₅, -SO₂-NH-CO-NH-D dans lequel D représente un radical phényle, pyridine ou pyrimidine, 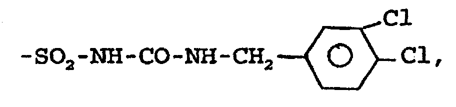 -SO₂-NH-CO-NH-CH₂-CH₂-CH₃, -SO₂-NH-CO-NH-CH=CH-CH₃, 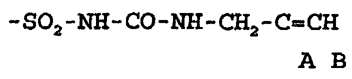 dans lequel A et B, identiques ou différents, sont choisis parmi l'atome d'hydrogène, les radicaux phényle, pyridyle et pyrimidyle, 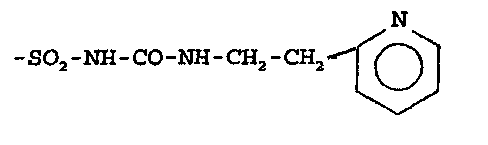 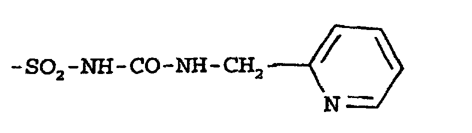 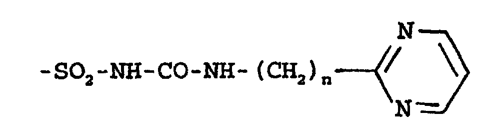 avec n = 1 ou 2

Dans les produits de formule (I) et dans ce qui suit :
- le terme radical alkyle linéaire ou ramifié désigne de préférence les radicaux méthyle, éthyle, propyle, isopropyle, butyle, isobutyle, sec-butyle et tert-butyle mais peut également représenter un radical pentyle ou hexyle et particulièrement isopentyle et isohexyle,
- le terme radical alkényle linéaire ou ramifié désigne de préférence un radical vinyle, allyle, 1-propényle, butényle et particulièrement 1-butényle, ou pentényle.
- le terme radical alkynyle linéaire ou ramifié désigne de préférence un radical éthynyle, propynyle, butynyle linéaire ou ramifié.
- le terme atome d'halogène désigne de préférence l'atome de chlore ou de brome, mais peut aussi représenter un atome de fluor ou d'iode,
- le terme radical alcoxy linéaire ou ramifié désigne de préférence les radicaux méthoxy, éthoxy, propoxy ou isopropoxy, mais peut aussi représenter un radical butoxy linéaire, secondaire ou tertiaire,
- le terme radical acyle désigne de préférence un radical ayant de 1 à 6 atomes de carbone tel que par exemple le radical formyle, acétyle, propionyle, butyryle ou benzoyle, mais également le radical pentanoyle, hexanoyle, acryloyle, crotonoyle ou carbamoyle,
- le terme amino substitué par un ou deux radicaux alkyle désigne de préférence des radicaux dans lesquels le ou les radicaux alkyle sont choisis parmi les radicaux alkyle tels que définis ci-dessus comme par exemple pour monoalkylamino, le radical méthylamino ou éthylamino, ou par exemple pour dialkylamino le radical diméthylamino ou encore méthyléthylamino,
- le terme radical acyloxy désigne par exemple un radical dans lequel le radical acyle a les valeurs indiquées ci-dessus et désigne de préférence un radical formyloxy, acétyloxy, propionyloxy, butyryloxy ou benzoyloxy,
- le terme radical haloalkyle désigne de préférence les radicaux dans lesquels le radical alkyle est tel que défini ci-dessus et est substitué par un ou plusieurs atomes d'halogène tel que défini ci-dessus par exemple bromoéthyle, trifluorométhyle, trifluoroéthyle ou encore pentafluoroéthyle,
- le terme radical alkylthio désigne de préférence les radicaux dans lesquels le radical alkyle est tel que défini ci-dessus, par exemple méthylthio ou éthylthio,
- le terme radical haloalkylthio désigne de préférence les radicaux dans lesquels le radical alkyle est tel que défini ci-dessus et est substitué par un ou plusieurs atomes d'halogène tel que défini ci-dessus, par exemple bromoéthylthio, trifluorométhylthio, trifluoroéthylthio ou encore pentafluoroéthylthio,
- le terme radical haloalcoxy désigne de préférence les radicaux dans lesquels le radical alcoxy est tel que défini ci-dessus et est substitué par un ou plusieurs atomes d'halogène tel que défini ci-dessus, par exemple bromoéthoxy, trifluorométhoxy, trifluoroéthoxy ou encore pentafluoroéthoxy,
- le terme radical carbamoyle désigne également les radicaux carbamoyle substitué par un groupe N-monoalkyl inférieur carbamoyle, tel que N-méthylcarbamoyle, N-éthylcarbamoyle, un groupe N,N-dialkyl inférieur carbamoyle, tel que N,N-diméthylcarbamoyle, N,N-diéthylcarbamoyle ; un groupe N-(hydroxyalkyl inférieur) carbamoyle, tel que N-(hydroxyméthyl) carbamoyle, N-(hydroxyéthyl) carbamoyle, un groupe carbamoylalkyle inférieur, tel que carbamoylméthyle, carbamoyléthyle,
- le terme radical phényle substitué par un radical alkylthio représente par exemple le radical benzylthio.
- les termes radical alkylthio, alkylsulfinyle et alkylsulfonyle désignent les radicaux dans lesquels le radical alkyle linéaire ou ramifié peut représenter, par exemple, les valeurs indiquées ci-dessus pour le radical alkyle ; ces radicaux représentent ainsi de préférence les radicaux méthylthio, hydroxyméthylthio, éthylthio, aminoéthylthio, méthylsulfinyle, éthylsulfinyle, méthylsulfonyle, éthylsulfonyle mais peut aussi représenter un radical propylthio, isopropylthio, butylthio, sec-butylthio, tert-butylthio, isopentylthio ou isohexylthio ou ces radicaux dans lesquels le radical thio est oxydé en radical sulfinyle ou sulfonyle.

Les radicaux carbamoyle et amino mentionnés plus haut et en particulier : les radicaux 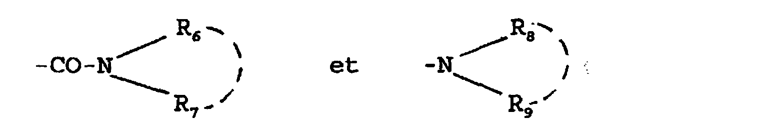 désignent des radicaux dans lesquels à l'atome d'azote sont liés deux radicaux, identiques ou différents, choisis parmi l'atome d'hydrogène pour donner le radical amino ; les radicaux alkyle tels que définis ci-dessus pour donner les radicaux monoalkyl- ou dialkylamino dans lesquels les radicaux alkyles linéaires ou ramifiés renferment de 1 à 6 atomes de carbone et en particulier des radicaux méthyle, éthyle, isopropyle, méthoxyméthyle, méthoxyéthyle, éthoxyéthyle ; les radicaux phényle, benzyle, phénéthyle, éventuellement substitués pour donner par exemple le radical phénylamino ou benzylamino.

Parmi les radicaux carbamoyle substitués on peut citer comme radical carbamoyle substitué le groupe N-monoalkyl inférieur carbamoyle, par exemple, N-méthylcarbamoyle, N-éthylcarbamoyle ; le groupe N,N-dialkyl inférieur carbamoyle, par exemple, N,N-diméthylcarbamoyle, N,N-diéthylcarbamoyle ; le groupe N-(hydroxyalkyl inférieur) carbamoyle, par exemple, N-(hydroxyméthyl) carbamoyle, N-(hydroxyéthyl) carbamoyle ; le groupe carbamoylalkyle inférieur, par exemple carbamoylméthyle, carbamoyléthyle ; phénylcarbamoyle ; pyridylcarbamoyle ; benzylcarbamoyle ; N-méthyl N-phénylcarbamoyle ; pyridylméthylcarbamoyle.

Le ou les radicaux carboxy des produits de formule (I) peuvent être salifiés ou estérifiés par les groupements divers connus de l'homme du métier parmi lesquels on peut citer, par exemple :
- parmi les composés de salification, des bases minérales telles que, par exemple, un sel de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxycarbonyle
ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxyméthyle, propionyloxyméthyle, méthylthiométhyle, diméthylaminoéthyle, benzyle ou phénéthyle.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acibdes alkylmonosulfoniques tels que par exemple l'acide méthanesulfonique, l'acide éthanesulfonique, l'acide propanesulfonique, les acides alkyldisulfoniques tels que par exemple l'acide méthanedisulfonique,l'acide alpha, bêta-éthanedisulfonique, les acides arylmonosulfoniques tels que l'acide benzènesulfonique et les acides aryldisulfoniques.

Parmi les produits préférés de l'invention, se trouvent en particulier les produits de formule (I) dans lesquels l'un de R₂ et R₃ représente un groupement soufré éventuellement oxydé tel que défini ci-dessus et l'autre de R₂ et R₃ représente de préférence un radical alkyle, alcoxy, carboxy libre salifié ou estérifié ou phényle éventuellement substitué par un ou plusieurs substituants ainsi qu'il est défini ci-dessus.

Parmi les produits préférés de l'invention, se trouvent tout particulièrement les produits de formule (I) dans lesquels R₂ représente un radical soufré.

Les produits de formule (I) représentent donc particulièrement les produits dans lesquels R₂ et R₃ ont les significations indiquées ci-dessus et tout particulièrement les produits dans lesquels R₂ représente un radical alkylthio éventuellement substitué tel que défini ci-dessus ou alcoxy tel que par exemple méthoxy et R₃ représente un radical carboxy libre, salifié ou estérifié, ou amidifié tel que notamment -COOH, -COO méthyl, -COO-éthyl, -CONH₂ ou

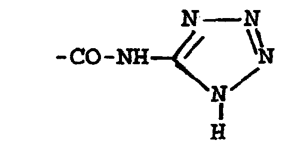

L'invention a plus particulièrement pour objet le procédé ci-dessus pour la préparation de produits de formule (I) répondant à la formule (I_{d}) : 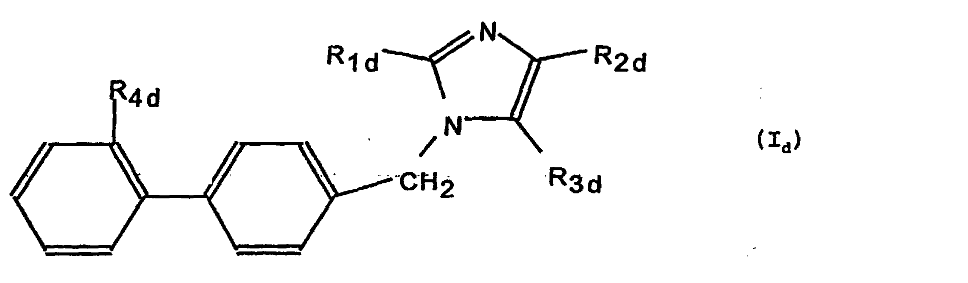 dans laquelle :
R_{1d} représente un radical alkyle renfermant au plus 4 atomes de carbone,
R_{3d} représente un radical :
   carboxy libre, salifié ou estérifié par un radical alkyle linéaire ou ramifié renfermant au plus 4 atomes de carbone, formyle, acyloxy, alkyle renfermant au plus 4 atomes de carbone éventuellement substitués par un radical hydroxyle,
R_{2d} représente un radical :
   phénylthio, phénylsulfonyle, phénylsulfinyle,
   alkylthio, alkylsulfonyle ou alkylsulfinyle,
   dans lesquels le radical alkyle renferme au plus 4 atomes de carbone,
et R_{4d} représente le radical -SO₂-NH₂, -SO₂-NH-CO-O-R_{10d}, -SO₂-N=CH-NR_{13d}, ou -SO₂-NH-CO-NH-R_{10d} dans lesquels R_{10d} et R_{13d} identiques ou différents, sont choisis parmi l'atome d'hydrogène, le radical méthyle, éthyle, n-propyle et propényle, lesdits produits de formule (I_{d}) étant sous toutes les formes isomères possibles racémiques, énantiomères et diastéréoisomères, ainsi que les sels d'addition avec les acides minéraux et organiques ou avec les bases minérales et organiques, desdits produits de formule (I_{d}), caractérisé en ce que l'on utilise pour leur préparation telle que définie ci-dessus, des produits de formules (II), (III) et (IV) dans lesquels R'₁, R'₂, R'₃ et R'₄ ont les valeurs indiquées ci-dessus respectivement pour R_{1d}, R_{2d}, R_{3d} et R_{4d} dans lesquelles les fonctions réactives sont éventuellement protégées.

L'invention a tout particulièrement pour objet le procédé ci-dessus, caractérisé en ce que l'on utilise au départ un produit de formule (II) dans laquelle R'₁ représente un radical alkyle renfermant au plus 4 atomes de carbone, R'₃ représente un radical : carboxy libre, salifié ou estérifié par un radical alkyle linéaire ou ramifié renfermant au plus 4 atomes de carbone, formyle, acyloxy, alkyle renfermant au plus 4 atomes de carbone éventuellement substitués par un radical hydroxyle,
R'₂ représente un radical :
phénylthio, phénylsulfonyle, phénylsulfinyle,
alkylthio, alkylsulfonyle ou alkylsulfinyle,
dans lesquels le radical alkyle renferme au plus 4 atomes de carbone, et les fonctions réactives sont éventuellement protégées et un produit de formule (V) dans laquelle R'₄ représente, le radical -SO₂-NH₂, -SO₂-NH-CO-O-R_{10d}, -SO₂-N=CH-NR_{13d}, ou -SO₂-MH-CO-NH-R_{10d} dans lesquels R_{10d} et R_{13d} identiques ou différents, sont choisis parmi l'atome d'hydrogène, le radical méthyle, éthyle, n-propyle et propényle, dans lesquels les fonctions réactives sont éventuellement protégées.

L'invention a tout particulièrement pour objet le procédé ci-dessus, caractérisé en ce que l'on utilise au départ un produit de formule (II) dans laquelle R'₃ représente un radical alcoxy ou carboxy libre, salifié ou estérifié,
et R'₂ représente un radical alkylthio ou phénylthio éventuellement oxydés sous forme de sulfoxyde ou de sulfone, ces radicaux alcoxy, alkylthio et phénylthio étant éventuellement substitués par un ou plusieurs radicaux choisis parmi les atomes d'halogène, les radicaux alkyl ou alcoxy renfermant au plus 4 atomes de carbone, trifluorométhyle, amino, mono ou dialkylamino, cyano, acyle, acyloxy ou phényl.

L'invention a pour objet particulièrement le procédé tel que défini ci-dessus, de préparation des produits de formule (I) répondant aux formules suivantes :
- l'acide 2-butyl 1-[[2'-carboxy (1,1'-biphényl)-4-yl] méthyl] 4-(phénylthio) 1H-imidazole-5-carboxylique
- l'acide 2-butyl 1-[[2'-carboxy (1,1'-biphényl)-4-yl] méthyl] 4-(méthylthio) 1H-imidazole-5-carboxylique
- l'acide 4'-[[2-butyl 4-(éthylthio) 5-(hydroxyméthyl) 1H-imidazol-1-yl] méthyl] (1,1'-biphényl)-2-carboxylique
- l'acide 2-butyl 1-[[2'-carboxy (1,1'-biphényl)-4-yl] méthyl] 4-(éthylsulfonyl) 1H-imidazole-5-carboxylique
- l'acide 2-butyl 1-[[2'-carboxy (1,1'-biphényl)-4-yl] méthyl] 4-(éthylsulfinyl) 1H-imidazole-5-carboxylique
- l'acide 2-butyl 1-[[2'-carboxy (1,1'-biphényl)-4-yl] méthyl] 4-(éthylthio) 1H-imidazole-5-carboxylique
- l'acide 2-butyl 1-[[2'-carboxy (1,1'-biphényl)-4-yl] méthyl] 4-(phénylsulfonyl) 1H-imidazole-5-carboxylique
- l'acide 2-butyl 1-[[2'-carboxy (1,1'-biphényl)-4-yl] méthyl] 4-(phénylsulfinyl) 1H-imidazole-5-carboxylique
- l'acide 2-butyl 1-[[2'-tétrazolyl (1,1'-biphényl)-4-yl] méthyl] 4-(méthylthio) 1H-imidazole-5-carboxylique,
- le 2-butyl 4-(méthylthio) 1-[(2'-((((propylamino) carbonyl) amino) sulfonyl) (1,1'-biphényl)-4-yl] méthyl]-1H-imidazole 5-carboxylate d'éthyle,
- l'acide 2-butyl 4-(méthylthio) 1-[[2'-((((propylamino) carbonyl) amino) sulfonyl) (1,1'-biphényl)-4-yl]méthyl]-1H-imidazole 5-carboxylique,
- l'acide 2-butyl 4-(méthylthio) 1-[[2'-((((propylamino) carbonyl) amino) sulfonyl) (1,1'-biphényl)-4-yl] méthyl]-1H-imidazole 5-carboxylique, di sel de potassium.

L'invention a plus particulièrement pour objet le procédé tel que défini ci-dessus, de préparation des produits de formule (I) répondant aux formules suivantes :
- le 2-butyl 4-(méthylthio) 1-[[2'-((((propylamino) carbonyl) amino) sulfonyl) (1,1'-biphényl)-4-yl] méthyl]-1H-imidazole 5-carboxylate d'éthyle,
- l'acide 2-butyl 4-(méthylthio) 1-[[2'-((((propylamino) carbonyl) amino) sulfonyl) (1,1'-biphényl)-4-yl] méthyl]-1H-imidazole 5-carboxylique,
- l'acide 2-butyl 4-(méthylthio) 1-[[2'-((((propylamino) carbonyl) amino) sulfonyl) (1,1'-biphényl)-4-yl] méthyl]-1H-imidazole 5-carboxylique, di sel de potassium.

Dans des conditions préférentielles de mise en oeuvre de l'invention, le produit de formule (III) est tel que Hal représente de préférence un atome de brome mais peut également représenter un atome de chlore ou d'iode.

La réaction du produit de formule (III) sur le produit de formule (II) peut être réalisée dans un solvant tel que par exemple le diméthylformamide, le tétrahydrofuranne, l'acétone, l'acétonitrile, la diméthylpropylurée, le diméthoxyéthane ou le diméthylsulfoxyde au reflux du solvant ou à la température ambiante, de préférence sous agitation ; la réaction est réalisée en présence d'une base telle que par exemple l'hydrure de sodium ou de potassium, le méthylate, l'éthylate ou le tert-butylate de sodium ou de potassium, ou encore et de préférence, le carbonate de sodium, de potassium ou de césium.

La réaction du produit de formule (IV) ainsi obtenu avec le composé de formule (V) tel que défini ci-dessus dans lequel X₄ représente de préférence un atome de brome, d'iode ou de chlore, peut être réalisée dans un solvant tel que par exemple un mélange de toluène et d'éthanol ou encore du diméthylformamide en présence d'une base faible telle que par exemple le bicarbonate de sodium, de potassium ou de césium, de préférence en présence d'un catalyseur tel que par exemple le tétrakis triphénylphosphine palladium ou un mélange de triphénylphosphine et de diacétate de palladium ou encore dans du tétrakis triphénylphosphine nickel.

Comme autres catalyseurs, on peut citer par exemple les complexes de nickel, Pd, Rh ou Pt et de préférence des complexes de palladium ; bis(dibenzylidène acétone) Pd en présence de PPh₃ ; tris(dibenzylidène acétone) Pd ; transbenzyl (chloro) bis(triphénylphosphine) palladium ; Pd(OAc)₂ tris furyl phosphine ; Pd(OAc)₂ triphényl phosphine ; tétrakis (triphényl phosphine) palladium ; 1,4 bis (diphényl phosphino) butane Pd, Cl, Br ou OAc ; 1,3 bis(diphényl phosphino) propane Pd, Cl, Br ou OAc ; 1,2 bis(diphényl phosphino) éthane Pd, Cl, Br ou OAc ; 1,1 bis(diphényl phosphino) ferrocene Pd, Cl, Br, ou OAc.

Dans la réaction du produit de formule (IV) avec le produit de formule (V), les solvants utilisés sont de préférence préalablement dégazés par exemple par barbotage d'argon.

Les diverses fonctions réactives que peuvent porter certains composés des réactions définies ci-dessus peuvent, si nécessaire, être protégées : il s'agit par exemple des radicaux hydroxyle, acyle, carboxy libres ou encore amino et monoalkylamino qui peuvent être protégés par les groupements protecteurs appropriés.

On trouvera une liste de différents groupements protecteurs utilisables par exemple dans le brevet BF 2 499 995.

La liste suivante, non exhaustive, d'exemples de protection de fonctions réactives peut être citée :
- les groupements hydroxyle peuvent être protégés par exemple par les radicaux alkyle tels que tert-butyle, triméthylsilyle, tert-butyldiméthylsilyle, méthoxyméthyle, tétrahydropyrannyle, benzyle ou acétyle,
- les groupements amino peuvent être protégés par exemple par les radicaux acétyle, trityle, benzyle, tert-butoxycarbonyle, phtalimido ou d'autres radicaux connus dans la chimie des peptides,
- les groupements acyles tel que le groupement formyle peuvent être protégés par exemple sous forme de cétals cycliques ou non cycliques tels que le diméthyl ou diéthylcétal ou l'éthylène dioxycétal,
- les fonctions acides des produits décrits ci-dessus peuvent être, si désiré, amidifiées par une amine primaire ou secondaire par exemple dans du chlorure de méthylène en présence, par exemple, de chlorhydrate de l-éthyl-3-(diméthylaminopropyl) carbodiimide à la température ambiante :
- les fonctions acides peuvent être protégées par exemple sous forme d'esters formés avec les esters facilement clivables tels que les esters benzyliques ou ter butyliques ou des esters connus dans la chimie des peptides.

Selon les valeurs de R'₁, R'₂, R'₃ et R'₄, les produits de formule (I') ainsi obtenus constituent ou non des produits de formule (I).

Lorsque les produits de formule (I') ne représentent pas des produits de formule (I), ils peuvent être soumis, si nécessaire et si désiré, aux réactions indiquées ci-dessous pour donner des produits de formule (I).

L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, formique ou trifluoroacétique ou encore par une hydrogénation catalytique.

Le groupement phtalimido peut être éliminé par l'hydrazine.

Les produits décrits ci-dessus peuvent, si désiré, faire l'objet de réactions de salification par exemple par un acide minéral ou organique selon les méthodes usuelles connues de l'homme du métier.

Les produits décrits ci-dessus peuvent, si désiré, faire l'objet, sur les éventuelles fonctions carboxy, de réactions de salification par une base minérale ou organique ou d'estérification : ces réactions d'estérification et de salification peuvent être réalisées selon les méthodes usuelles connues de l'homme du métier.

Les éventuelles transformations de fonctions ester en fonction acide des produits décrits ci-dessus peuvent être, si désiré, réalisées dans les conditions usuelles connues de l'homme du métier notamment par hydrolyse acide ou alcaline par exemple par de la soude ou de la potasse en milieu alcoolique tel que, par exemple, dans du méthanol ou encore par de l'acide chlorhydrique ou sulfurique.

Les éventuelles fonctions cyano des produits décrits ci-dessus peuvent être, si désiré, transformées en fonction acide dans les conditions usuelles connues de l'homme du métier par exemple par une double hydrolyse réalisée en milieu acide tel que par exemple dans un mélange d'acide sulfurique, d'acide acétique glacial et d'eau, ces trois composés étant de préférence en proportions égales, ou encore dans un mélange de soude, d'éthanol et d'eau au reflux.

Les éventuelles fonctions carboxy libre ou estérifié des produits décrits ci-dessus peuvent être, si désiré, réduites en fonction alcool par les méthodes connues de l'homme de métier : pour les fonctions carboxy estérifié, on peut utiliser notamment l'hydrure de lithium et d'aluminium dans un solvant tel que par exemple le tétrahydrofuranne ou encore le dioxane ou l'éther éthylique.

Pour les fonctions carboxy libre, on peut utiliser notamment par l'hydrure de bore.

Les éventuelles fonctions alcoxy telles que notamment méthoxy des produits décrits ci-dessus peuvent être, si désiré, transformées en fonction hydroxyle dans les conditions usuelles connues de l'homme du métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou par de l'acide acétique au reflux.

Les éventuels groupements comprenant un atome de soufre des produits décrits ci-dessus peuvent être, si désiré, transformés en fonction sulfoxyde ou sulfone correspondantes dans les conditions usuelles connues de l'homme du métier telles que par exemple au moyen de peracides comme par exemple l'acide peracétique ou l'acide métachloroperbenzoïque ou encore par l'ozone, l'oxone, le périodate de sodium dans un solvant tel que par exemple le chlorure de méthylène ou le dioxane à la température ambiante.

La fonction sulfoxyde peut être obtenue par un mélange équimolaire du produit renfermant un groupement alkylthio ou arylthio et du réactif tel que notamment un peracide.

La fonction sulfone peut être obtenue par un mélange du produit renfermant un groupement alkylthio ou arylthio avec un excès du réactif tel que notamment un peracide.

Les éventuelles fonctions alcool des produits décrits ci-dessus peuvent être, si désiré, transformées en fonction aldéhyde ou acide par oxydation dans les conditions usuelles connues de l'homme du métier telles que par exemple par action de l'oxyde de manganèse pour obtenir les aldéhydes ou du réactif de Jones pour accéder aux acides.

Les éventuelles fonctions nitrile des produits décrits ci-dessus peuvent être, si désiré, transformées en tétrazole dans les conditions usuelles connues de l'homme du métier telles que par exemple par cycloaddition d'un azidure métallique tel que par exemple un azidure de trialkylétain sur la fonction nitrile ainsi qu'il est indiqué dans la méthode décrite dans l'article référencé comme suit : J. Organometallic Chemistry., 33, 337 (1971) KOZIMA S.& coll.

Les éventuelles formes optiquement actives des produits de formule (I) peuvent être préparées par dédoublement des racémiques selon les méthodes usuelles connues de l'homme du métier.

Les réactions de transformation de radical formyle en radical carbamoyle et de radical carbamoyle en radical nitrile sont réalisées selon les conditions usuelles connues de l'homme du métier.

Les produits de formule (I) sont connus et décrits notamment dans les demandes de brevets européens n° 0 465 368 et 0 503 162.

Les produits de formule (I) préparés selon le procédé tel que défini ci-dessus, ainsi que leurs sels d'addition avec les acides présentent d'intéressantes propriétés pharmacologiques.

Les produits sont doués de propriétés antagonistes pour le récepteur à l'angiotensine II et sont ainsi notamment inhibiteurs des effets de l'angiotensine II, en particulier de l'effet vasoconstricteur et également de l'effet trophique au niveau des myocytes.

Ces propriétés justifient l'application en thérapeutique des produits de formule (I) préparés selon le procédé tel que défini ci-dessus à titre de médicaments, lesdits produits de formule (I) étant sous toutes les formes isomères possibles racémiques ou optiquement actives, ainsi que les sels d'addition avec les acides minéraux ou organiques pharmaceutiquement acceptables desdits produits de formule (I).

Les produits de formule (I) préparés selon le procédé défini ci-dessus ainsi que leurs sels d'addition avec les acides minéraux ou organiques pharmaceutiquement acceptables, peuvent être utilisés notamment à titre de médicaments, dans le traitement de l'hypertension artérielle, des insuffisances cardiaques, des insuffisances rénales et dans la prévention des resténoses post-angioplastie.

Ils peuvent également être utilisés dans le traitement de certains désordres gastro-intestinaux, gynécologiques et en particulier pour un effet relaxant au niveau de l'utérus, sous forme de compositions pharmaceutiques renfermant à titre de principe actif l'un au moins des médicaments tels que définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 1 à 100 mg par jour chez l'adulte, par voie orale.

Certains produits de départ de formule (II) sont connus et peuvent être préparés par exemple comme indiqué dans le brevet européen EP 168 950.

Les produits de départ de formule (II) peuvent en particulier être préparés selon le procédé caractérisé en ce que l'on soumet un composé de formule (IIₐ) : 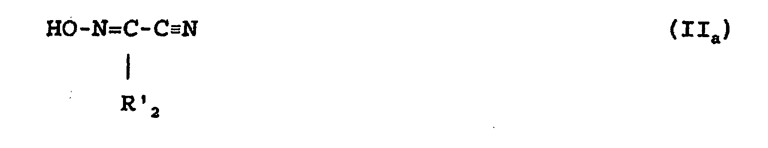 dans laquelle R'₂ a la signification indiquée ci-dessùs, à l'action d'un agent réducteur, pour obtenir l'amine correspondante de formule (II_{b}) : 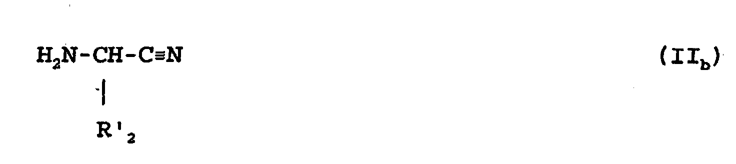 dans laquelle R'₂ a la signification indiquée précédemment, produit de formule (II_{b}) que l'on soumet à l'action d'un composé de formule (II_{c}) : 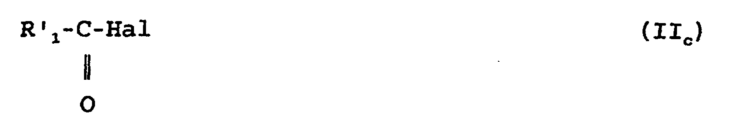 dans laquelle R'₁ a la signification indiquée ci-dessus, et Hal représente un atome d'halogène, pour obtenir un produit de formule (II_{d}) : 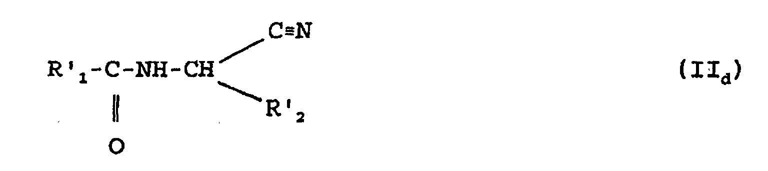 dans laquelle R'₁ et R'₂ ont les significations indiquées précédemment, que l'on fait réagir avec un composé de formule (IIₑ) :

R'₃-YH (IIe)

dans laquelle R'₃ a la signification indiquée ci-dessus, et Y représente un atome de soufre ou d'oxygène pour obtenir un produit de formule (II_{f}) : 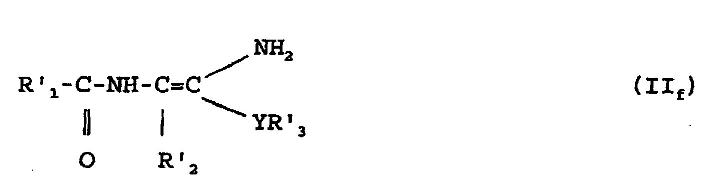 dans laquelle R'₁, R'₂, R'₃ et Y ont les significations indiquées précédemment, que l'on soumet à une réaction de cyclisation pour obtenir un produit de formule (II), produit de formule (II) que l'on soumet, si désiré et si nécessaire, à l'une ou plusieurs des réactions suivantes, dans un ordre quelconque :
a) une réaction d'élimination des groupements protecteurs que peuvent porter les fonctions réactives protégées,
b) une réaction de salification par un acide minéral ou organique ou par une base pour obtenir le sel correspondant,
c) une réaction d'estérification de fonction acide,
d) une réaction de saponification de fonction ester en fonction acide,
e) une réaction de transformation de la fonction cyano en fonction acide,
f) une réaction de réduction de la fonction carboxy en fonction alcool,
g) une réaction de transformation de fonction alcoxy en fonction hydroxyle,
h) une réaction d'oxydation du groupement comprenant un atome de soufre en fonction sulfoxyde ou sulfone correspondant,
i) une réaction d'oxydation de fonction alcool en fonction aldéhyde ou acide,
j) une réaction de transformation de fonction nitrile en tétrazole,
k) une réaction de dédoublement des formes racémiques en produits dédoublés,
l) une réaction de transformation du radical carboxy en radical carbamoyle,
m) une réaction de transformation du radical carbamoyle en radical nitrile,
lesdits produits de formule (II) ainsi obtenus étant sous toutes les formes isomères possibles racémiques, énantiomères et diastéréoisomères.

Dans des conditions préférentielles de mise en oeuvre de l'invention, le procédé ci-dessus est réalisé de la manière suivante :
- la réduction de l'oxime de formule (IIₐ) pour donner le composé de formule (II_{b}) peut être réalisée selon les méthodes usuelles connues de l'homme du métier telles que par exemple par un amalgame d'aluminium préparé dans les conditions usuelles telles que par exemple par action du chlorure de mercure sur de l'aluminium : la réaction est réalisée dans un solvant tel que par exemple le tétrahydrofuranne ou le toluène, de préférence à une température d'environ 50°C ;
- l'addition du produit de formule (II_{c}) dans laquelle W représente un atome de brome ou de préférence un atome de chlore, sur l'amine de formule (II_{b}) peut être réalisée selon les méthodes connues de l'homme du métier, par exemple en présence d'une base telle que la pyridine ou la triéthylamine; la réaction est réalisée de préférence à une température d'environ 0°C.

On peut également obtenir le composé de formule (II_{d}) en soumettant le composé de formule (IIₐ) à une réaction de réduction et d'acylation en présence d'un anhydride tel que par exemple l'anhydride acétique, butyrique ou valérique, par hydrogénation en présence de palladium ou de zinc ou encore de dithionite de sodium.
- l'addition du dérivé soufré de formule (IIₑ) sur l'amide de formule (II_{d}) est réalisée, par exemple, par mise en solution de l'amide de formule (II_{d}) dans un solvant par exemple un alcool tel que l'éthanol ou le méthanol, puis addition successive d'une base telle que par exemple la triéthylamine et du composé de formule (IIₑ) de préférence sous agitation et à la température ambiante,
- la réaction de cyclisation du composé de formule (II_{f}) peut être réalisée dans un solvant tel que, par exemple, le dichlorométhane, le dichloroéthane ou encore le chloroforme : la réaction peut être réalisée par exemple en présence de pentachlorure de phosphore dissous au préalable dans du dichorométhane à une température d'environ -78°C en présence d'une base telle que par exemple la pyridine ou la diméthylaminopyridine : la réaction peut être réalisée sous agitation à la température ambiante.

Le produit de formule (II) ainsi obtenu peut être soumis à l'une ou plusieurs des réactions indiquées ci-dessus, ces réactions pouvant être réalisées dans les mêmes conditions que-celles définies ci-dessus pour les produits de formule (I).

Le composé de formule (IIₐ) peut être, par exemple, l'éthylisonitrosocyanoacétate que l'on peut trouver, par exemple, sous forme de produit commmercialisé par LANCASTER sous la référence 8930.

Le composé de formule (III) tel que défini ci-dessus, peut être obtenu en faisant réagir le composé en équilibre sous sa forme trimère, de formule (VI) : 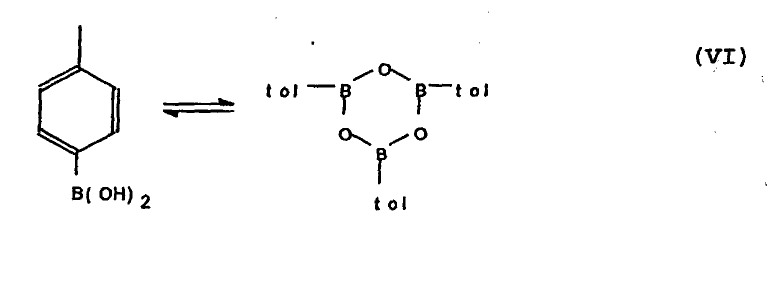 dans laquelle :
B est défini comme précédemment et tol représente le radical tolyle avec un alcool approprié tel que par exemple le méthanol, l'éthanol ou le butanol, ou un diol tel que par exemple le propanediol, l'éthylèneglycol ou diméthylpropanediol,
pour obtenir un composé de formule (VIII) : 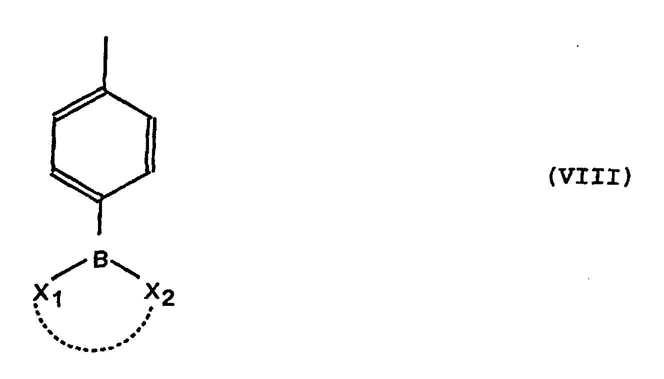 dans laquelle B, X₁ et X₂ ont les significations indiquées ci-dessus,
produit de formule (VIII) que l'on fait réagir avec un agent d'halogènation, pour obtenir le produit de formule (III).

On peut encore indiquer le procédé de préparation du produit de formule (I) tel que défini ci-dessus, caractérisé en ce que le produit de formule (IV) est obtenu en faisant réagir le composé de formule (VI) tel que défini ci-dessus avec un agent d'halogénation comme indiqué dans la référence H.R. SNYDER et Coll. J. Ann. Chem. Soc. 80, 835 (1958) pour obtenir le composé en équilibre avec sa forme trimère, de formule (VI') : 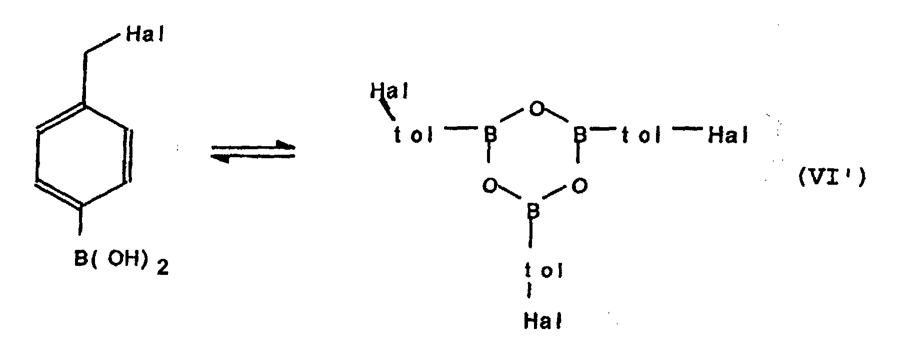 dans laquelle B et tol ont les significations indiquées ci-dessus, et Hal représente un atome d'halogène, de préférence un atome de brome,
composé de formule (VI') que l'on fait réagir avec le composé de formule (II) pour obtenir le produit de formule (IV), puis poursuit la synthèse comme indiqué ci-dessus pour obtenir le produit de formule (I).

De telles réactions peuvent être réalisées dans les conditions indiquées ci-dessus et ainsi qu'il est décrit ci-après dans la préparation des exemples.

Ainsi qu'il est indiqué ci-dessus, le procédé de préparation des produits de formule (I) telle que définie ci-dessus, peut donc se faire par réaction du produit de formule (II) : 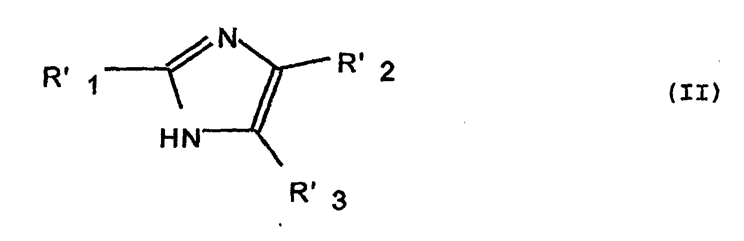 dans laquelle R'₁, R'₂ et R'₃ ont les significations indiquées ci-dessus, le produit de formule (III) répondant à la formule (IIIₐ) : 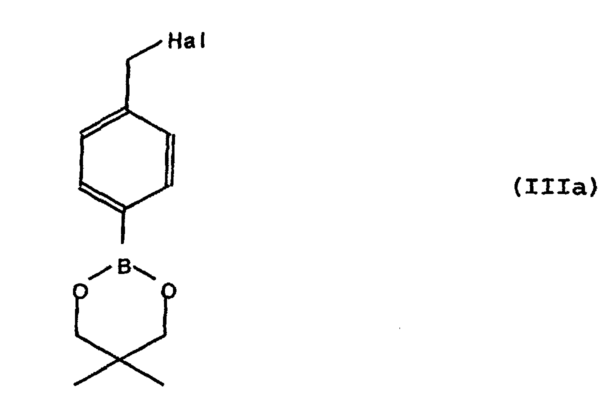 dans laquelle Hal et B ont les significations indiquées ci-dessus, pour donner le produit de formule (IV) répondant à la formule (IVₐ) : 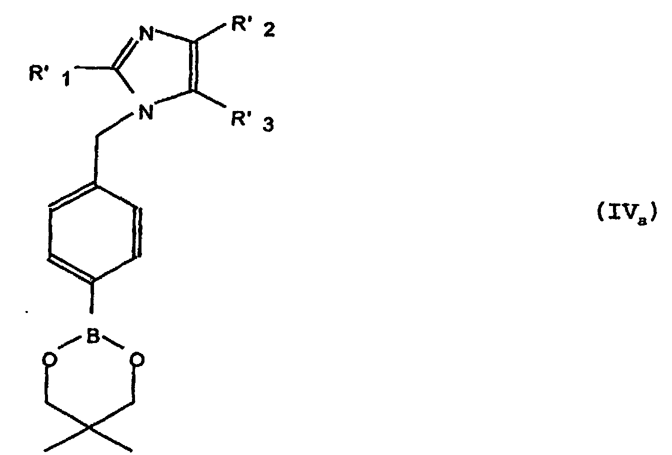 dans laquelle R'₁, R'₂, R'₃ et B ont les significations indiquées ci-dessus, que l'on fait réagir avec un composé de formule (Vₐ) 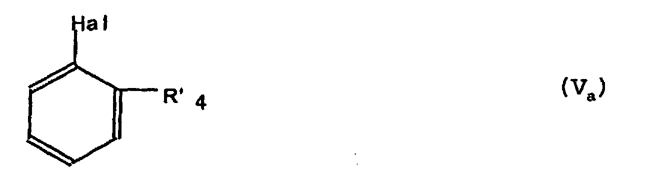 dans laquelle Hal et R'₄ ont les significations indiquées ci-dessus.

Un exemple d'une telle préparation d'un produit de formule (I) telle que définie ci-dessus est donnée ci-après à l'exemple 1.

Dans le procédé décrit ci-dessus, on peut faire réagir le produit de formule (II) telle que définie ci-dessus avec le produit de formule (III) répondant à la formule (III_{b}) : 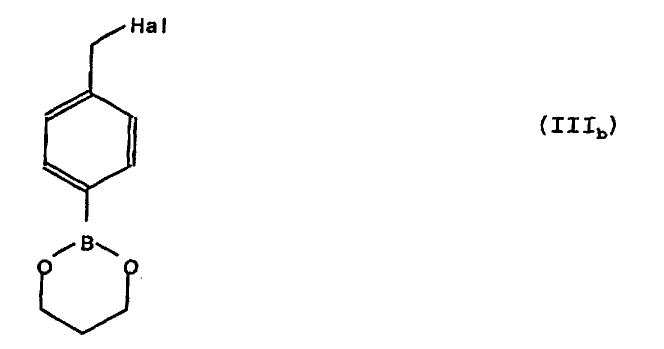 dans laquelle Hal et B ont les significations indiquées ci-dessus, pour obtenir un produit de formule (IV) répondant à la formule (IV_{b}) ou (IV'_{b}) : 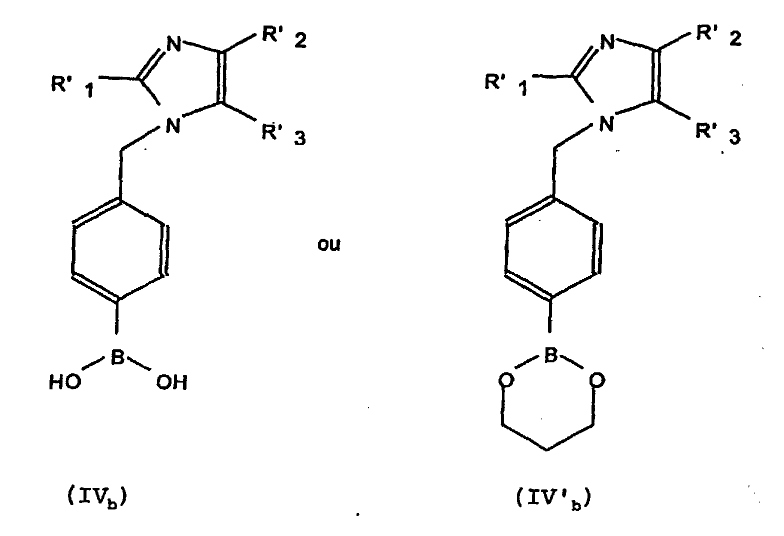 dans lesquelles R'₁, R'₂, R'₃ et B ont les significations indiquées ci-dessus, produit de formule (IV_{b}) ou (IV'_{b}) que l'on fait réagir avec un produit de formule (V) telle que définie ci-dessus.

Un exemple d'une telle préparation d'un produit de formule (I) telle que définie ci-dessus, est donné ci-après à l'exemple 2.

Un tel produit de formule (I) peut être soumis, si nécessaire et si désiré, à diverses réactions pour donner d'autres produits de formule (I), ainsi qu'il est indiqué ci-dessus notamment à une réaction de saponification. De même, les produits de formule (IV) et à titre d'exemple les produits de formule (IVₐ), (IV_{b}) ou (IV'_{b}), peuvent également être soumis à diverses réactions pour donner d'autres produits de formule (IV).

La présente invention a également pour objet à titre de produits industriels nouveaux et notamment à titre de produits intermédiaires nécessaires au procédé de préparation des produits de formule (I), les composés de formules (IV) et (V) telles que définies ci-dessus.

Certains produits de formule (III) sont également nouveaux et constituent à ce titre un objet de l'invention.

La présente invention a tout particulièrement pour objet, à titre de produits industriels nouveaux, les produits :
**1** - 2-iodobenzène sulfonamide
**2 -** 2-iodo N-[(propylamino) carbonyl] benzène sulfonamide
**4a -** 2-[4-(bromométhyl) phényl] 5,5-diméthyl 1,3,2-dioxaborolane
**4b** - 2-[4-(bromométhyl) phényl] 1,3,2-dioxaborolane
**5 -** 1-[(4-boronophényl) méthyl] 2-butyl 4-(méthylthio) 1H-imidazole 5-carboxylate d'éthyle
**6a -** 2-butyl 1-[4-(5,5-diméthyl 1,3,2-dioxaborolan-2-yl) phénylméthyl] 4-(méthylthio) 1H-imidazole 5-carboxylate d'éthyle
**6b -** 2-butyl 1-[4-(1,3,2-dioxaborolan 2-yl) phényl méthyl] 4-(méthylthio) 1H-imidazole 5-carboxylate d'éthyle
**7 -** (T-4)((4-((2-butyl 4-(méthylthio) 5-(éthoxycarbonyl) 1H-imidazole 1-yl((méthyl) phényl) ((2,2'-(méthylimino) bis-(éthanolato)) (2-)-N,O,O')-bore
**8 -** 2-butyl 1-((4-(1,3,2-benzodioxaborol-2-yl) phényl) méthyl) 4-(méthylthio) 1H-imidazole 5-carboxylate d'éthyle
**9a -** 2-butyl 1-((4-(4,4,5,5-tétraméthyl 1,3,2-dioxolaborolan-2-yl) phényl) méthyl) 4-(méthylthio) 1H-imidazole 5-carboxylate d'éthyle
**9b -** 2-butyl 1-((4-(1,3,2-dioxolaborolan-2-yl) phényl) méthyl) 4-(méthylthio) 1H-imidazole 5-carboxylate d'éthyle.

Ces produits 1, 2, 3, 4a, 4b, 5, 6a, 6b, 7, 8, 9a et 9b correspondent aux schémas suivants des produits 1 à 9 : 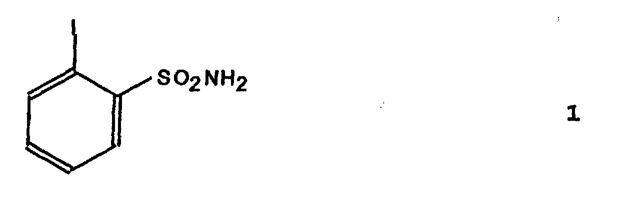 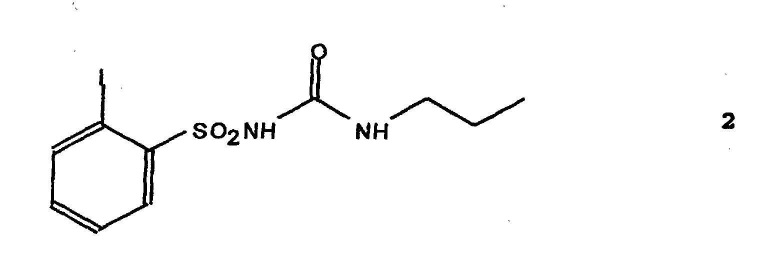 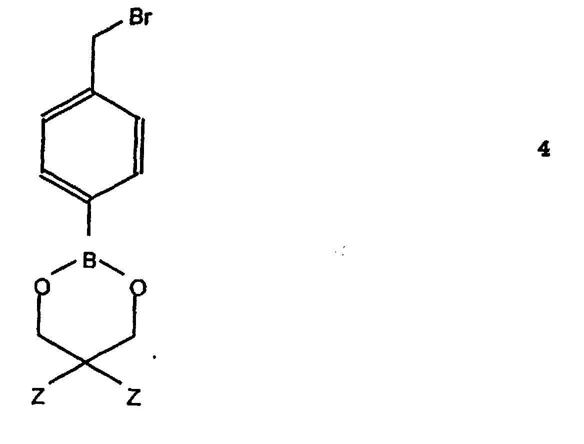 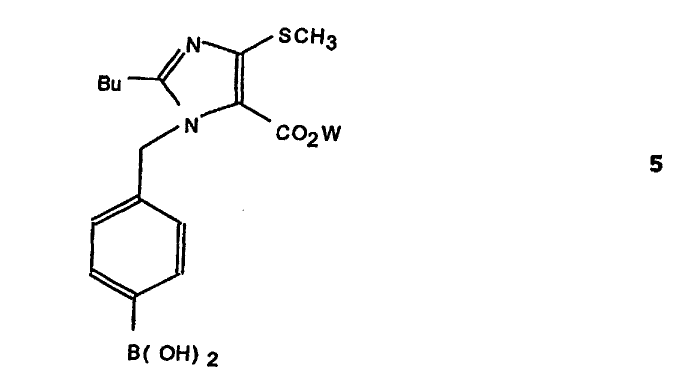 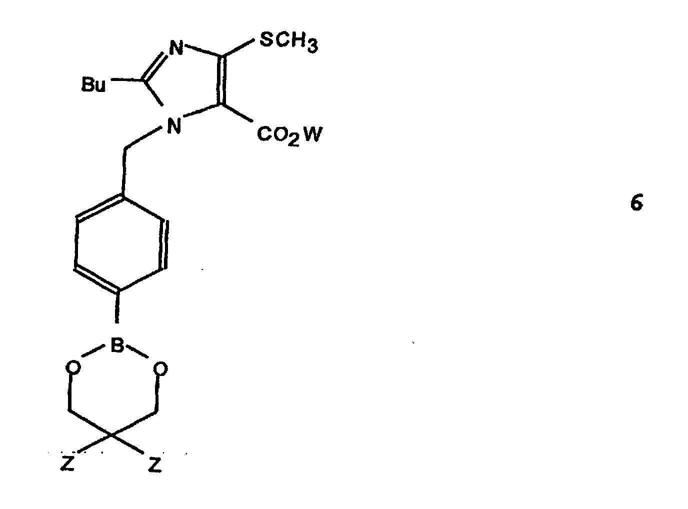 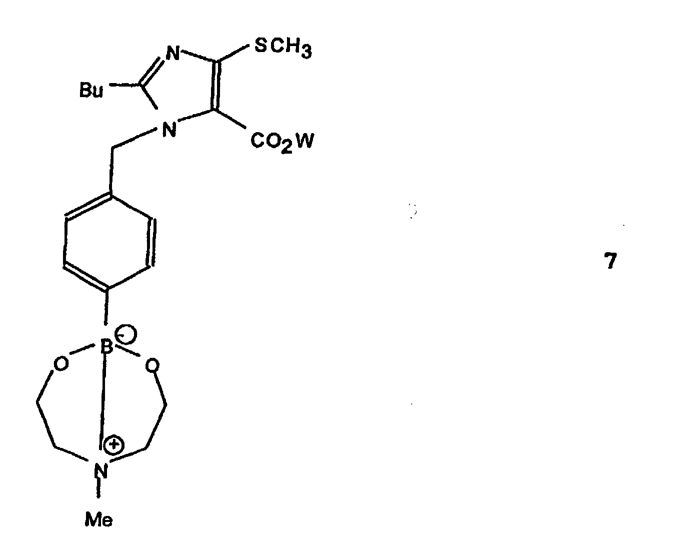 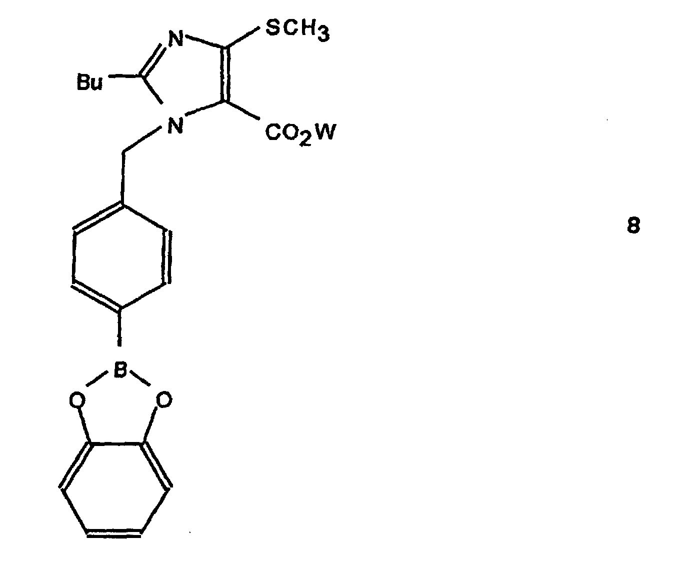 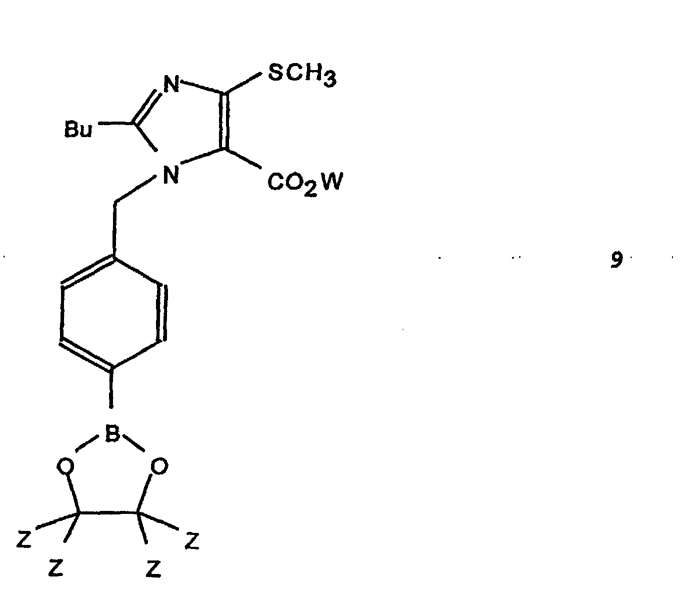 dans lesquels Z et W, identiques ou différents, représentent un atome d'hydrogène ou un radical alkyle et notamment méthyle ou éthyle et les produits 4, 6 et 9 tels que définis ci-dessus sont respectivement appelés 4a, 6a et 9a lorsque Z représente un radical méthyle et 4b, 6b et 9b lorsque Z représente un atome d'hydrogène.

Des méthodes de préparations des composés ci-dessus et de certains de leurs homologues sont fournies ci-après dans la partie expérimentale.

Les exemples suivants de préparation de produits de formule (I) illustrent l'invention sans toutefois la limiter.

### Préparation 1 : 2-iodobenzène sulfonamide (produit 1)

On chauffe à 60°C, 3,5 g de α-aminobenzène sulfonamide et 25 ml d'acide sulfurique concentré à 98 %. On ajoute 20 g de glace et ajoute à 0-5°C, une solution de 1,45 g de nitrite de sodium dans 4 ml d'eau. On agite 3 heures à température inférieure à 10°C, puis on ajoute à 5-10°C, une solution de 3,75 g de iodure de potassium dans 25 ml d'eau.

On laisse sous agitation 19 heures et ajoute 50 ml d'eau, on filtre, lave à l'eau puis reprend avec 50 ml d'acétate d'éthyle, lave avec une solution 0,2N de thiosulfate de sodium, puis à l'eau, concentre et obtient 4 g de produit attendu.

### Résultats analytiques

F = 197-198°C
IR (nujol)
3360, 3255, 1562 cm⁻¹
Spectre de masse M⁺ 283
RMN : CDCl₃
5,17 (sl, NH₂) ; 7,23 et 7,52 (td, aromatiques, 2H) ; 8,08 et 8,20 (dd, aromatiques, 2H).

### Préparation 2 : 2-iodo N-[(propylamino) carbonyl] benzène sulfonamide (produit 2)

On mélange 4 g de iodo benzène sulfonamide et 40 ml d'acétone. On ajoute 3,92 g de carbonate de potassium. On porte au reflux, ajoute 1,46 ml de n-propyl isocyanate et maintient 2 heures au reflux. On concentre, ajoute 200 ml d'eau, puis, en refroidissant le mélange réactionnel à environ 0-5°C, ajoute une solution d'acide chlorhydrique 2N, jusqu'à pH 3. On recristallise dans l'acétone-éther isopropylique et obtient 4,9 g de produit attendu.

### Résultats analytiques

F = 211-212°C
IR (nujol)
3406, 3368, 1715, 1565, 1539 cm⁻¹
Spectre de masse M⁺ 368
RMN : CDCl₃
0,82 (tJ = 7,5, CH₃, 3H) ; 1,45 (m, CH₂, 2H) ; 3,14 (m, CH₂, 2H) ; 6,39 (t, CONH, 1H) ; 7,29 (dt, J=1,5, aromatiques) ; 7,54 (td, J=8,15, aromatiques) ; 8,13 (m, aromatiques) ; 7,59 (s, SO₂NH, 1H).

La préparation des produits 6a, 6b, 7, 8, 9a et 9b décrite dans la partie expérimentale, peut être schématisée comme indiqué ci-après.

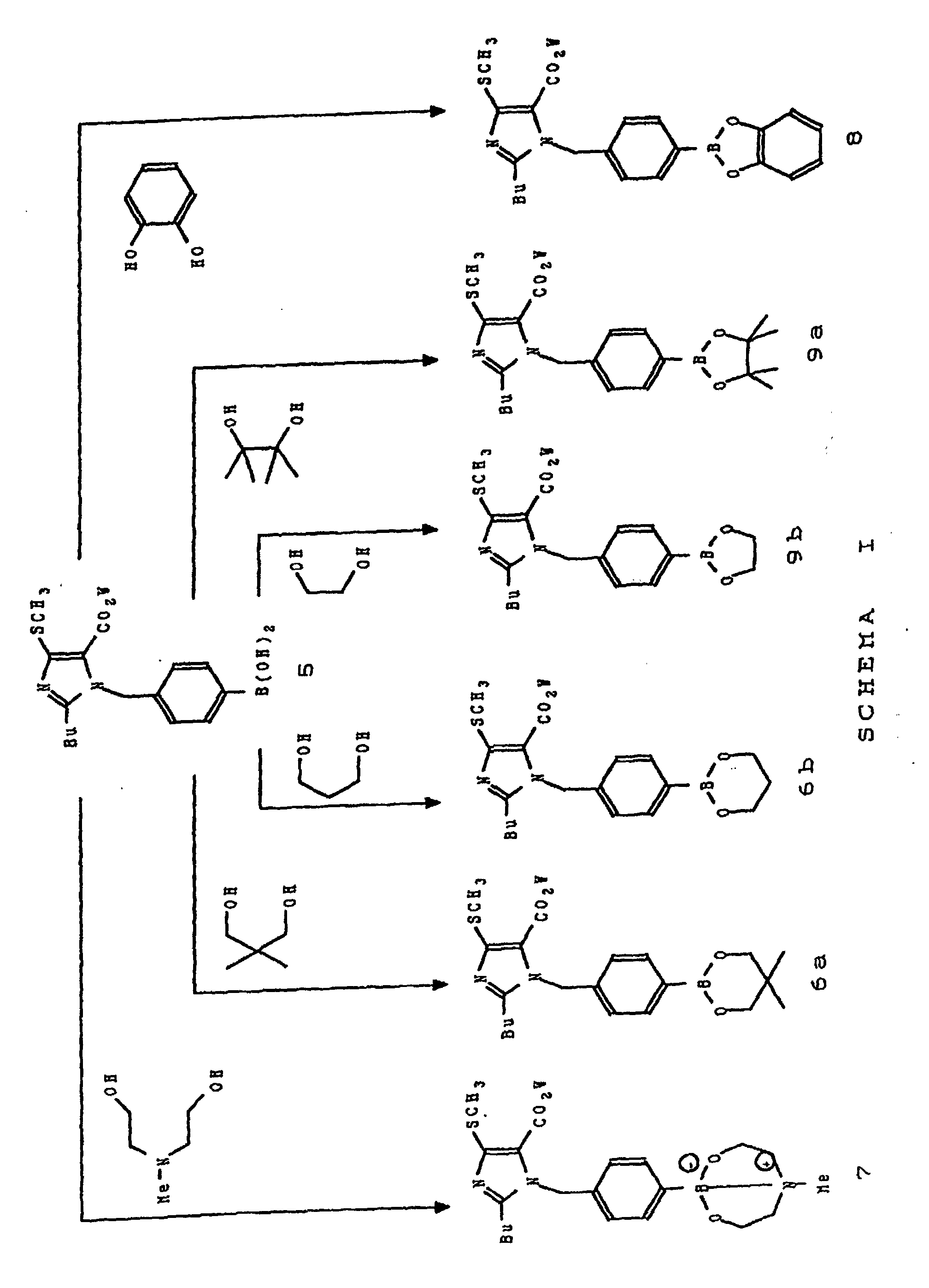

### Préparation 3 : 1-[(4-boronophényl) méthyl] 2-butyl 4-(méthylthio) 1H-imidazole 5-carboxylate d'éthyle (produit 5)

### Méthode 1 :

A un mélange de 0,484 g de 2-butyl 4-(méthylthio) 1H-imidazole 5-carboxylate d'éthyle, 5 ml de diméthylformamide et 0,552 g de carbonate de potassium, on ajoute 0,423 g d'acide 4-bromo méthyl phényl boronique (préparé selon la méthode décrite par Snyder et Coll. JACS 80, 835 (1958)). Après 48 h d'agitation à température ambiante le mélange réactionnel est versé dans l'eau glacée. On agite 15 minutes. On filtre, lave à l'eau et recristallise dans un mélange de cyclohexane et éther isopropylique pour obtenir 0,450 g du produit attendu.

### Méthode 2 :

Dans un mélange de 9,44 g 2-butyl 4-(méthylthio) 1H-imidazole 5-carboxylate d'éthyle, 62 ml de diméthylformamide anhydre et 10,78 g de carbonate de potassium, on ajoute une solution de 11 g de 2-(4-(bromo méthyl) phényl) 1,3,2-dioxaborolane dans 66 ml de diméthylformamide. Après 48 h d'agitation à température ambiante le mélange réactionnel est versé dans l'eau glacée et acidifié à pH 2 avec de l'acide chlorohydrique 2N. On filtre, lave à l'eau et sèche pour obtenir 10,45 g du produit attendu.
F = 169-170°C
IR (nujol)
1693, 1610, 1555, 1513 cm⁻¹
Spectre de masse 1074 (forme trimère)
RMM : CDCl₃
Un mélange de monomère et de trimère dédoublements - 3/4-1/4 de signaux 0,87 et 0,86 (t CH₃, 3H) ; 1,32 (m, CH₂, 2H) ; 1,30 (t, CO₂CH₂CH₃, 3H) ; 1,64 (m, CH₂, 2H) ; 2,60 (m, CH₂, 2H) ; 2,61 et 2,63 (S, SCH₃, 3H) 4,25 (q dédoublé, CO₂CH₂CH₃, 2H) ; 5,55 et 5,60 (s, N-CH₂-Ph, 2H) ; 7,00 et 7,68 (dl, aromatiques, 2H) ; 7,11 et 8,12 (dl, aromatiques, 2H) ; 4,93 (m, large - 0,2H, mobile).

### Préparation 4 : 2-butyl 1-[4-(1,3,2-dioxaborolan-2-yl) phényl méthyl] 4-(méthylthio) 1H-imidazole 5-carboxylate d'éthyle (produit 6b)

On agite au reflux, pendant 4 heures, en éliminant l'eau formée, un mélange de 1,292 g de 1-[(4-boronophényl) méthyl] 2-butyl 4-(méthylthio) 1H-imidazole 5-carboxylate d'éthyle, obtenu comme indiqué à la préparation 3, 25 cm³ de toluène et 0,710 cm³ de 1,3 propanediol. On évapore à sec sous pression réduite, cristallise le résidu par chaud et froid dans l'heptane, filtre, lave et obtient 1,12 g de produit recherché.
RMN : CDCl₃ (250 MHz) ppm
0,86 (t, 3H) : CH₃ nBu ; 1,30 (m, 2H)-1,60 (m, 2H)-2,58 (t, 2H) : les CH₂ nBu ; 1,28 (t, 3H)-4,23 (q, 2H) : -COOEt ; 2,04 (m, 2H) : O-CH₂-CH₂-CH₂-O ; 4,14 (t, 4H) : -O-CH₂-CH₂-CH₂-O-O- ; 2,58 (s, 3H) : : SCH₃ ; 5,52 (s, 2H) : Ar-CH₂-imidazole ; 6,94 et 7,68 (2D, 4H) : aromatiques.

### Préparation 5 : 2-butyl 1-[4-(5,5-diméthyl 1,3,2-dioxaborolan-2-yl) phénylméthyl] 4-(méthylthio) 1H-imidazole 5-carboxylate d'éthyle (produit 6a)

On opère comme à la préparation 4 en utilisant 250 mg de 1-[(4-boronophényl) méthyl] 2-butyl 4-(méthylthio) 1H-imidazole 5-carboxylate d'éthyle et 70 mg de 2,2'-diméthyl 1,3-propanediol, on obtient 200 mg du produit recherché.

### Préparation 6 : (T-4)-((4-((2-butyl 4-(méthylthio) 5-(éthoxycarbonyl)-1H-imidazol-1-yl) méthyl) phényl) ((2,2'-(méthylimino) bis (éthanolato)) (2-)-N,O,O') -bore (produit 7)

On opère comme à la préparation 4, en utilisant 2 g de 1-[(4-boronophényl) méthyl] 2-butyl 4-(méthylthio) 1H-imidazole 5-carboxylate d'éthyle, 30 cm³ de cyclohexane et 10 cm³ d'acétate d'éthyle puis après dissolution à chaud, 0,611 cm³ de N-méthyldiéthanolamine. On obtient après concentration 2,17 g de produit recherché.
RMN : CDCl₃ (250 MHz) ppm
0,85 (t, 3H) : CH₃ nBu ; 1,3 (t, 3H) : CH₃ de -COOEt ; 1,3 (m, 2H)-1,62 (q, 2H) : CH₂ en 3 et CH₂ en 2 nBu 2,30 (s, 3H) ;-N⁺-CH₃ ; 2,60 (s, 2H et t, 2H) : S-CH₃ et CH₂ en 1 nBu ; 2,98 et 3,20 (m, 4H) : CH₂-N⁺ **≡** ; 4,15 (m, 4H) CH₂-B⁻≡ ; 4,27 (q, 2H) : CH₂ de COOEt ; 5,50 (s, 2H) : Ar-CH₂-imidazole ; 6,9 et 7,5 (2d, 4H) aromatiques.

### Préparation 7 : 2-butyl 1-((4-(1,3,2-benzodioxaborol-2-yl) phényl) méthyl) 4-(méthylthio) 1H-imidazole 5-carboxylate d'éthyle (produit 8)

On opère comme à la préparation 4, en utilisant 1 g de 1-[(4-boronophényl) méthyl] 2-butyl 4-(méthylthio) 1H-imidazole 5-carboxylate d'éthyle et 0,293 g de catéchol. On obtient 1,16 g du produit recherché.
RMN : CDCl₃ (200 MHz) ppm
0,87 (t, 3H) : CH₃ de nBu ; 1,35 (m, 2H) : CH₂ en 3 de nBu ; 1,65 (m, 2H) : CH₂ en 2 de nBu ; 2,63 (t, 2H) : CH₂ en 1 de nBu ; 1,30 (t, 3H) : CH₃ de CO₂Et ; 4,25 (q, 2H) : CH₂ de CO₂Et ; 2,63 (s, 3H) : SCH₃ ; 5,6 (s, 2H) : CH₂ benzyl ; 7,1 et 8,05 (2d) : aromatiques ; 7,15 et 7,3 : catéchol.

### Préparation 8 : 2-butyl 1-((4-(4,4,5,5-tétraméthyl 1,3,2-dioxolaborolan-2-yl) phényl) méthyl) 4-(méthylthio) 1H-imidazale 5-carboxylate d'éthyle (produit 9a)

On opère comme à la préparation 4, à partir de 0,5 g de 1-[(4-boronophényl) méthyl] 2-butyl 4-(méthylthio) 1H-imidazole 5-carboxylate d'éthyle en utilisant 0,189 g de pinacol.
On obtient 0,511 g de produit recherché.
RMN : CDCl₃ (250 MHz) ppm
0,87 (t, 3H) : CH₃ de nBu ; 1,33 (m, 2H) : CH₂ en 3 de nBu ; 1,63 (m, 2H) : CH₂ en 2 de nBu ; 2,53 (t, 2H) : CH₂ en 1 de nBu ; 1,33 (s, 12H) : 4 CH₃-C- ; 1,23 (t, 3H) : CO₂Et ; 4,23 (q, 2H) : CO₂Et ; 2,61 (s, 3H) : S-CH₃ ; 5,53 (s) : N-CH₂-Ar ; 6,37 et 7,73 (2d,4H) : aromatiques.

### Préparation 9: 2-butyl 1-((4-(1,3,2-dioxolaborolan-2-yl) phényl) méthyl) 4-(méthylthio) 1H-imidazole 5-carboxylate d'éthyle (produit 9b)

On opère comme à la préparation 4, à partir de 0,2 g de 1-[(4-boronophényl) méthyl] 2-butyl 4-(méthylthio) 1H-imidazole 5-carboxylate d'éthyle en utilisant 99 mg d'éthylène glycol. On obtient 0,21 g de produit attendu.
RMN : CDCl₃ (200 MHz) ppm
0,9 (t, 3H) : CH₃ de nBu ; 1,35 (m, 2H) : CH₂ en 3 de nBu ; 1,65 (m, 2H) : CH₂ en 2 de nBu ; 2,63 (t, 2H) : CH₂ en 1 de nBu ; 1,30 (t, 3H) : CO₂Et ; 4,25 (q, 2H) : CO₂Et ; 2,63 (s, 3H) : S-CH₃ ; 5,60 (s, 2H) : Ar-CH₂-imidazole ; 4,45 (s,4H) : 2CH₂-O ; 7,02 et 7,8 (2d) aromatiques.

### Préparation 10 : 2-bromo N-[(propylamino) carbonyl] benzène sulfonamide

On chauffe au reflux 15 g de 2-bromobenzène sulfonamide dans 150 cm³ d'acétone, ajoute 17,6 g de carbonate de potassium puis 6,6 cm³ de N-propylisocyanate, agite 2 heures 30 au reflux, refroidit à 0°C et acidifie jusqu'à pH 5. On obtient 18,35 g de produit attendu (F=218-219°C).
RMN : CDCl₃ ppm
0,84 (t, 3H) : CH₃ ; 1,45 (m, 2H), 3,07 (q, 2H) : les CH₂ ; 6,08 (l, 1H) : NH ; 7,5 (m, 2H), 7,71 (dd, 1H), 8,25 (dd, 1H) : les aromatiques ; 10,00 (1, 1H) : NH.

### EXEMPLE 1 : Acide 2-butyl 4H-(méthylthio) 1-[[2'-((((propylamino) carbonyl) amino) sulfonyl) 1,1'(biphényl) 4-yl] méthyl] 1H-imidazole 5-carboxylique di sel de potassium

### Stade A : 2-[(4-bromométhyl) phényl] ] 5,5-diméthyl 1,3,2-dioxaborolane

On chauffe 4 heures au reflux, en éliminant l'eau formée, 3,4 g d'acide 4-méthyl phényl boronique (ou sa forme trimère) et 2,6 g de 2,2-diméthyl propane 1,3-diol dans 50 cm³ de cyclohexane. On ajoute 4,45 g de N-bromosuccinimide et 100 mg d'azobis isobutyronitrile. On maintient 4 heures au reflux, refroidit, filtre, lave avec du cyclohexane et obtient 7 g de produit recherché. F = 110°C.
RMN : CDCl₃ ppm
1,01 (s, 6H) : (CH₃)₂-C ; 3,75 (s, 4H) : CH₂-O-B ; 4,52 (s, 2H) : CH₂Br ; 6,95 (d, 2H), 7,72 (d, 2H) : aromatiques.

### Stade B : 2-butyl 1-[4-(5,5-diméthyl 1,3,2-dioxaborolan-2-yl) phénylméthyl] 4-(méthylthio) 1H-imidazole 5-carboxylate d'éthyle

On agite pendant 30 minutes un mélange de 2-butyl 4-(méthylthio) 1H-imidazole 5-carboxylate d'éthyle, 65 cm³ de diméthylformamide et 3,75 g de carbonate de potassium puis on ajoute 8 g du produit obtenu au stade A en solution dans 32 cm³ de diméthylformamide. On agite 48 heures à température ambiante. On coule dans l'eau, extrait avec de l'acétate d'éthyle, sèche et évapore à sec sous pression réduite. Après empâtage dans un mélange de cyclohexane-acétate d'éthyle (8-2), on obtient 5,67 g du produit recherché (F=145-146°C).
RMN : CDCl₃ ppm
0,86 (t, 3H) : CH₃ de nBu ; 1,29 (t, 3H) : CH₃ de CO₂Et ; 1,34 (m, 2H), 1,62 (m, 2H), 2,60 (d, 2H) : les CH₂ de nBu ; 2,61 (s, 3H) : S-CH₃ ; 4,23 (q, 2H) : CH₂ de CO₂Et ; 5,52 (s, 2H) : CH₂ benzyle ; 6,96 (d, 2H) et 7,71 (d, 2H) : les aromatiques.

### Stade C : 2-butyl 4H-(méthylthio) 1-[[2'-((((propylamino) carbonyl) amino) sulfonyl) 1,1'(biphényl) 4-yl] méthyl] 1H-imidazole 5-carboxylate d'éthyle

On agite 24 heures au reflux un mélange de 62 mg de 2-bromo N-[(propylamino) carbonyl] benzène sulfonamide, obtenu selon la préparation 10, 4 cm³ de toluène, 0,193 cm³ d'une solution 2N de carbonate de sodium dans l'eau, 7 mg de tétrakis (triphénylphosphine) palladium, 1,6 cm³ d'éthanol et 85,5 mg du produit obtenu au stade B et 0,4 cm³ de toluène. On ajoute un peu de toluène et acidifie à pH 2 avec de l'acide chlorhydrique 2N. On extrait avec du chlorure de méthylène, lave à l'eau, sèche, évapore à sec et recueille 165 mg de produit que l'on chromatographie sur silice en éluant avec toluène-dioxane-acide acétique (95-4-1), on obtient ainsi 75 mg de produit recherché (F=182°C).
RMN : CDCl₃ ppm
0,72 (t, 3H) : CH₃ de nBu ; 1,67 (m, 2H) : CH₂ ; 3,03 (q, 2H) : CH₂-NH ; 6,01 (s, 1H) : SO₂NH ; 6,11 (t, 1H) : CONH ; 7,25 (d), 7,64 (t), 7,53 (t), 8,12 (d) : les aromatiques.

### Stade D : Acide 2-butyl 4H-(méthylthio) 1-[[2'-((((propylamino) carbonyl) amino) sulfonyl) 1,1'(biphényl) 4-yl] méthyl] 1H-imidazole 5-carboxylique di sel de potassium

A une solution de 2 g de produit obtenu comme au stade C dans 40 cm³ d'éthanol, on ajoute à 0°C, 2,3 cm³ d'une solution de potasse 6N. On laisse revenir à température ambiante. Après 72 heures, on essore le précipité et lave avec 4 cm³ d'éthanol puis avec 4 cm³ d'acétate d'éthyle. On obtient, après séchage, 2,04 g de produit recherché F> à 260°C.

| Analyse pour : C₂₆H₃₀K₂N₄O₅S₂ | | | | |
|---|---|---|---|---|
| | C | H | N | S |
| % calculés | 50,30 | 4,87 | 9,02 | 10,33 |
| % trouvés | 50,5 | 4,9 | 9,0 | 10,3 |

### EXEMPLE 2a : Acide 2-butyl 4H-(méthylthio) 1-[[2'-((((propyl amino) carbonyl) amino) sulfonyl) 1,1'(biphényl) 4-yl] méthyl] 1H-imidazole 5-carboxylique di sel de potassium

### Stade A : 2-[(4-bromométhyl) phényl] 1,3,2-dioxaborolane

On opère comme au stade A de l'exemple 1, à partir de 21,06 g d'acide 4-méthyl phényl boronique, en utilisant 17,4 cm³ de triméthylène glycol, 250 cm³ de cyclohexane, 440 mg d'asabis isobutyronitrile et 32,03 g de N-bromosuccinimide. On obtient 31 g du produit recherché (F= 108-109°C)
RMN (CDCl₃) ppm
2,05 (m, 2H) : CH₂ central ; 4,14 (t, 4H) : B-O-CH₂ ; 4,49 (s, 2H) : CH₂-Br ; 7,27 (d, 2H) et 7,74 (d, 2H) : aromatiques.

### Stade B : 2-butyl 1-[4-(1,3,2-dioxaborolan 2-yl) phényl méthyl] 4-(méthylthio) 1H-imidazole 5-carboxylate d'éthyle

On agite 30 minutes à 20-22°C un mélange de 34,5 g de 2-butyl 4-(méthylthio) 1H-imidazole 5-carboxylate d'éthyle, 59 g de carbonate de potassium, 3,82 g de bromure de tétrabutylammonium et 312 cm³ d'acétone. On ajoute ensuite 33,6 g du produit obtenu au stade A ci-dessus. On agite 24 heures, filtre et concentre à sec sous pression réduite. Après cristallisation par chaud et froid dans 59 cm³ d'éthanol, on obtient 20,3 g du produit recherché (F=120-121°C).
RMN (CDCl₃) ppm
0,86 (t, 3H) : CH₃ ; 1,28 (t, 3H) : CH₃ de CO₂Et ; 1,30 (m, 2H) : CH₂ ; 1,60 (m, 2H) : CH₂ ; 2,58 (t, 2H) : CH₂-C=N ; 2,55 (s, 3H) : S-CH₃ ; 2,04 (m, 2H) : CH₂ central ; 4,14 (t, 4H) : BO-CH₂ ; 4,23 (t, 2H) : CH₂ de CO₂Et ; 5,52 (s, 2H) : CH₂-N ; 6,94 (d, 2H) et 7,68 (d, 2H) : aromatiques.

### Stade C : 2-butyl 4H-(méthylthio) 1-[[2'-((((propylamino) carbonyl) amino) sulfonyl) 1,1'(biphényl) 4-yl] méthyl] 1H-imidazole 5-carboxylate d'éthyle

A un mélange agité 15 minutes de 264 mg du produit obtenu à la préparation 10 avec 1,3 cm³ de toluène, 1,027 cm³ d'une solution 2N de carbonate de sodium, 97,8 mg de bromure de potassium et 17,3 mg de galvinoxyl, on ajoute 12,9 mg de triphénylphosphine et 5,5 mg d'acétate de palladium. On agite 15 minutes et ajoute une solution de 400 mg du produit obtenu au stade B ci-dessus dans 1,3 cm³ toluène et 5,3 cm³ d'éthanol. On agite 24 heures au reflux. Après dilution avec du toluène et acidification par ajout de 1,47 cm³ d'acide chlorhydrique 2N, on extrait avec du chlorure de méthylène, sèche et évapore à sec sous pression réduite. On chromatographie le résidu sur silice (éluant cyclohexane/n-chlorobutane/isopropanol 75-25-5) et obtient 0,25 g du produit recherché identique au produit obtenu au stade C de l'exemple 1.

### Stade D : Acide 2-butyl 4H-(méthylthio) 1-[[2'-((((propylamino) carbonyl) amino) sulfonyl) 1,1'(biphényl) 4-yl] méthyl] 1H-imidazole 5-carboxylique di sel de potassium

On opère comme au stade D de l'exemple 1.

### EXEMPLE 2b : Acide 2-butyl 4H-(méthylthio) 1-[[2'-((((propylamino) carbonyl) amino) sulfonyl) 1,1'(biphényl) 4-yl] méthyl] 1H-imidazole 5-carboxylique di sel de potassium

On procède comme à l'exemple 2a ; on utilise au stade C à la place de 12,9 mg de triphénylphosphine, 10,1 mg de 1,3 bis diphényl phosphinopropane. On obtient ainsi 49,6 mg de produit obtenu au stade C de l'exemple 2a que l'on traite ainsi qu'il est indiqué au stade D de l'exemple 1 pour obtenir le produit attendu.

### EXEMPLE 3 : 2-butyl 4H-(méthylthio) 1-[[2'-((((propylamino) carbonyl) amino) sulfonyl) 1,1'(biphényl) 4-yl] méthyl] 1H-imidazole 5-carboxylate d'éthyle

En opérant comme au stade C de l'exemple 1, en utilisant 0,0824 g de 2-iodo N-propylamino carbonyl benzène sulfonamide obtenu selon la préparation 2, et 109 mg du produit obtenu à la préparation 4, on obtient le produit attendu.

### EXEMPLE 4 : 2-butyl 4H-(méthylthio) 1-[[2'-((((propylamino) carbonyl) amino) sulfonyl) 1,1'(biphényl) 4-yl] méthyl] 1H-imidazole 5-carboxylate d'éthyle

En opérant comme au stade C de l'exemple 2, en utilisant 828 mg de 2-iodo N-propylamino carbonyl sulfonamide et 1,2 g du produit obtenu à la préparation 5, on obtient 905 mg du produit recherché.

### EXEMPLE 5 : 2-butyl 4H-(méthylthio) 1-[[2'-((((propylamino) carbonyl) amino) sulfonyl) 1,1'(biphényl) 4-yl] méthyl] 1H-imidazole 5-carboxylate d'éthyle

On opère comme au stade C de l'exemple 1, à partir de 442 mg du produit obtenu à la préparation 6, en utilisant 264 mg du produit obtenu à la préparation *10,* on obtient ainsi 152 mg du produit recherché.

### EXEMPLE 6 : 2-butyl 4H-(méthylthio) 1-[[2'-((((propylamino) carbonyl) amino) sulfonyl) 1,1'(biphényl) 4-yl] méthyl] 1H-imidazole 5-carboxylate d'éthyle

On opère comme au stade C de l'exemple 2, à partir de 1 g du produit obtenu à la préparation 3, en utilisant 722 mg du produit obtenu à la préparation 10, on obtient ainsi 646 mg de produit recherché.

### EXEMPLE 7 : 2-butyl 4H- (méthylthio) 1-[[2' - ((((propylamino) carbonyl) amino) sulfonyl) 1,1'(biphényl) 4-yl] méthyl] 1H-imidazole 5-carboxylate d'éthyle

On opère comme à l'exemple 6, à partir de 1 g du produit obtenu à la préparation 3 et 828 mg de 2-iodo N-[(propylamino) carbonyl] benzène sulfonamide obtenu comme à la préparation 2 et obtient 571 mg du produit recherché.

### EXEMPLE 8 : 2-butyl 4H-(méthylthio) 1-[[2'-((((propylamino) carbonyl) amino) sulfonyl) 1,1'(biphényl) 4-yl] méthyl] 1H-imidazole 5-carboxylate d'éthyle

On opère comme au stade C de l'exemple 2 en utilisant à la place du produit obtenu au stade B de l'exemple 2, 432,8 mg du produit obtenu à la préparation 7 et obtient ainsi 121 mg de produit attendu.

### EXEMPLE 9 : 2-butyl 4H-(méthylthio) 1-[[2'-((((propylamino) carbonyl) amino) sulfonyl) 1,1'(biphényl) 4-yl] méthyl] 1H-imidazole 5-carboxylate d'éthyle

On opère comme au stade C de l'exemple 2 en utilisant à la place du produit obtenu au stade B de l'exemple 2, 387 mg du produit obtenu à la préparation 9 et obtient ainsi 220,5 mg de produit attendu.

### EXEMPLE 10 : 2-butyl 4H-(méthylthio) 1-[[2'-((((propylamino) carbonyl) amino) sulfonyl) 1,1'(biphényl) 4-yl] méthyl] 1H-imidazole 5-carboxylate d'éthyle

On opère comme au stade C de l'exemple 2 en utilisant à la place du produit obtenu au stade B de l'exemple 2, 440 mg du produit obtenu à la préparation 8 et obtient ainsi 172 mg de produit attendu.

### EXEMPLE 11 : composition pharmaceutique

On a préparé des comprimés répondant à la formule suivante :

| | |
|---|---|
| Produit de l'exemple 1 | 50 mg |
| Excipient pour un comprimé terminé à | 200 mg |
| (détail de l'excipient : lactose, talc, amidon, stéarate de magnésium). | |

On peut citer notamment les produits suivants qui peuvent être obtenus par le procédé tel que défini ci-dessus, objet de l'invention : 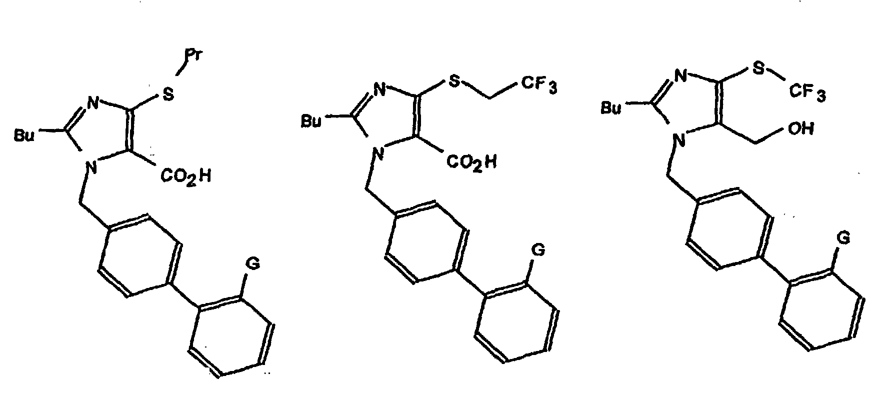 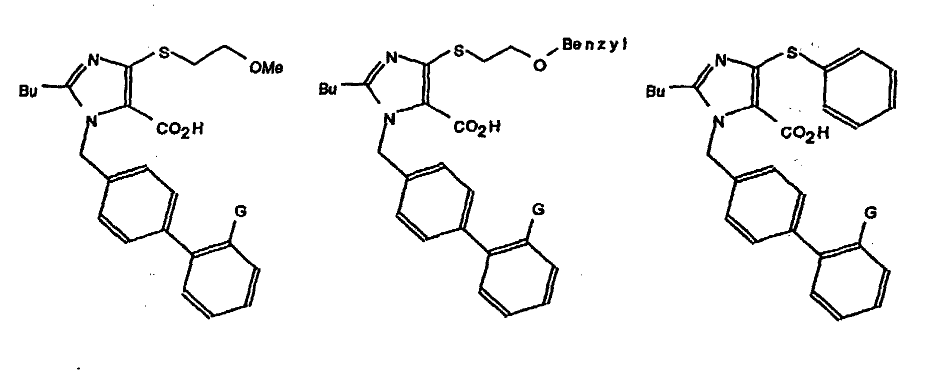 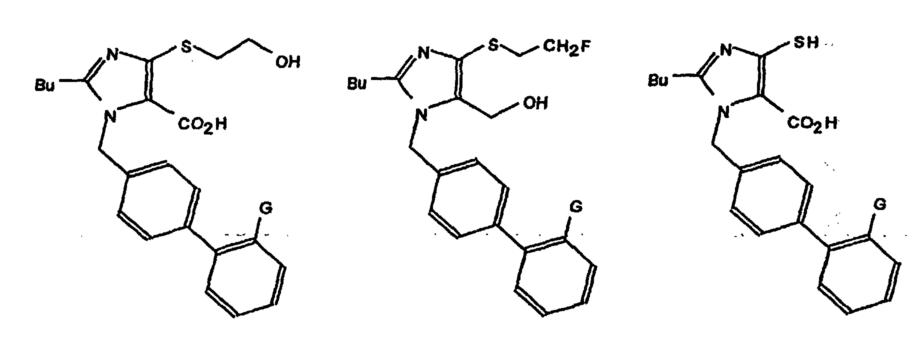 dans lesquels G représente de préférence
-SO₂-NH-CO-NH-CH₂-CH₂-CH₃ ou -SO₂-NH-CO₂-alkyle dans lequel alkyle représente de préférence un radical méthyle, éthyle, propyle ou butyle.

## Revendications

1. Procédé de préparation de produits de formule (I) : 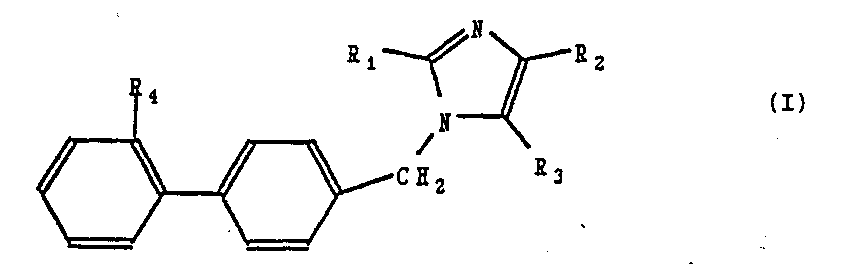 dans laquelle :
R₁ représente un radical alkyle renfermant au plus 4 atomes de carbone,
R₃ représente un atome d'hydrogène, un radical formyle, acyloxy, alkyle ou alcoxy éventuellement substitué, ou un radical carboxy libre, salifié ou estérifié par un radical alkyle,
R₂ représente un radical phénylthio, phénylsulfonyle, phénylsulfinyle, alkylthio, alkylsulfonyle ou alkylsulfinyle, éventuellement substitué, tel que dans tous ces radicaux que peuvent représenter R₂ et R₃, les radicaux alkyle, alcoxy renfermant au plus 6 atomes de carbone, et les radicaux phényle sont éventuellement substitués par un ou plusieurs radicaux choisis parmi les atomes d'halogène et les radicaux hydroxyle, trifluorométhyle, acyloxy, carboxy libre salifié ou estérifié par un radical alkyle, phényle, pyridyle, tétrazolyle, alkyle et alcoxy renfermant au plus 4 atomes de carbone et eux-mêmes éventuellement substitués par un radical alcoxy renfermant au plus 4 atomes de carbone,
R₄ représente le radical cyano, carboxy libre, salifié ou estérifié par un radical alkyle, le radical -SO₂-X-R₁₀ dans lequel X représente les radicaux -NH-, -NH-CO-, -NH-CO-O-, -N=CH-N-R13
-NH-CO-NH- ou une simple liaison et R₁₀ et R₁₃ identiques ou différents, représentent un atome d'hydrogène, un radical méthyle, éthyle, propyle, vinyle, allyle, pyridyle, phényle, benzyle, nitropyridyle, pyrimidyle, tétrazolyle, diazolyle, pipéridinyle, alkylpipéridinyle, thiazolyle, alkylthiazolyle, tétrahydrofuranyle, méthyltétrahydrofuranyle,
lesdits produits de formule (I) étant sous toutes les formes isomères racémiques, énantiomères et diastéréoisomères possibles, ainsi que les sels d'addition avec les acides minéraux et organiques ou avec les bases minérales et organiques desdits produits de formule (I), **caractérisé en ce que** l'on fait réagir un composé de formule (II) : 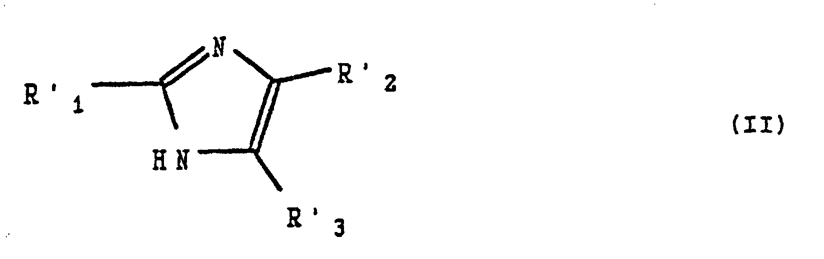 dans laquelle R'₁, R'₂ et R'₃ ont les significations indiquées ci-dessus respectivement pour R₁, R₂ et R₃ et dans lesquelles les éventuelles fonctions réactives sont éventuellement protégées, avec un composé de formule (III) : 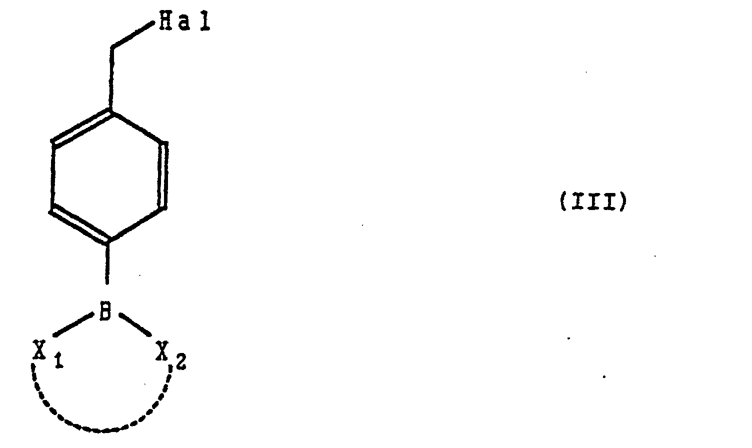 dans laquelle Hal représente un atome d'halogène, B représente un atome de bore et X₁, X₂ sont tels que :
ou bien X₁ et X₂ identiques ou différents représentent un radical hydroxyle, alkyle ou alcoxy renfermant au plus 6 atomes de carbone, un radical phényle ou phénoxy,
ou bien X₁ avec X₂ forment avec l'atome de bore auquel ils sont liés un cycle choisi parmi 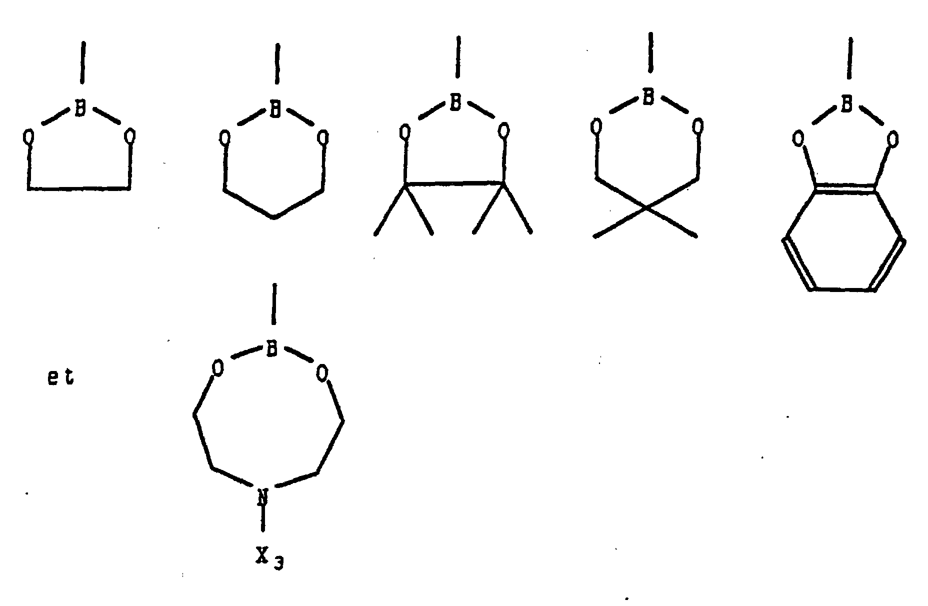 X₃ représentant un atome d'hydrogène ou un radical alkyle renfermant au plus 4 atomes de carbone, pour obtenir un produit de formule (IV) : 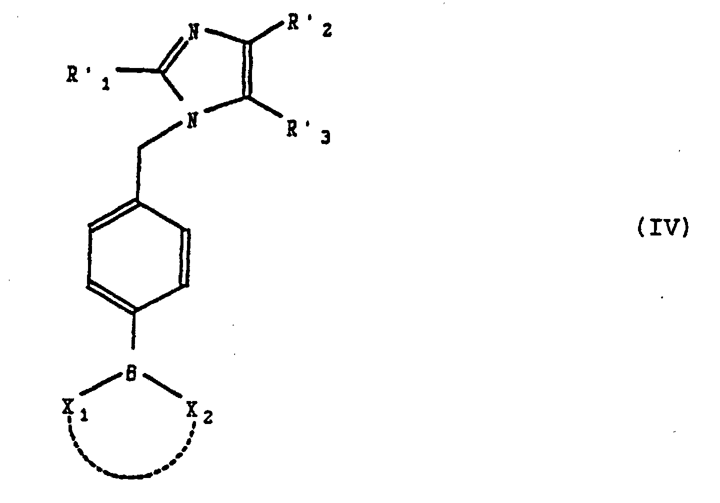 dans laquelle R'₁, R'₂, R'₃, X₁, X₂ et B ont les significations indiquées ci-dessus, que l'on fait réagir avec un produit de formule (V) : 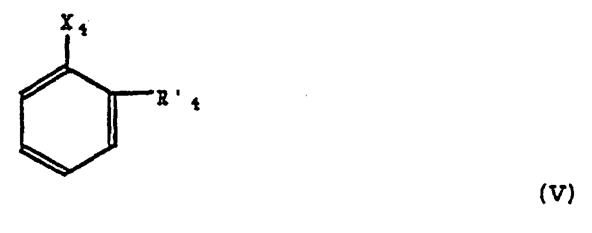 dans lequel X₄ représente un atome d'halogène, un radical alcoxy, triflate ou -O-SO₂F et R'₄ a la signification indiquée ci-dessus pour R₄ dans laquelle les éventuelles fonctions réactives sont éventuellement protégées, pour obtenir un produit de formule (I') : 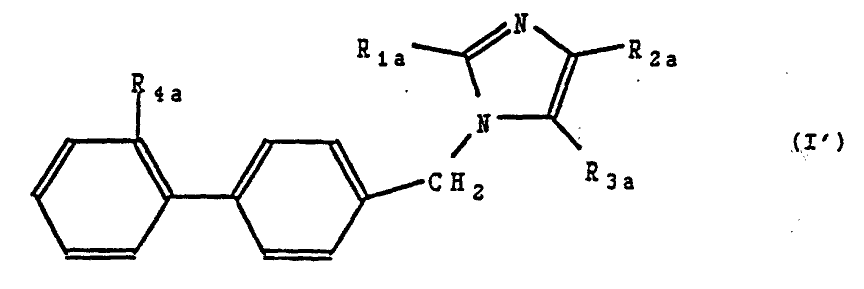 produit de formule (I') que l'on soumet, si désiré et si nécessaire, à l'une ou plusieurs des réactions suivantes, dans un ordre quelconque :
a) une réaction d'élimination des groupements protecteurs que peuvent porter les fonctions réactives protégées,
b) une réaction de salification par un acide minéral ou organique ou par une base,
c) une réaction d'estérification de fonction acide,
d) une réaction de saponification de fonction ester,
e) une réaction de transformation de la fonction cyano en fonction acide,
f) une réaction de réduction de la fonction carboxy en fonction alcool,
g) une réaction de transformation de fonction alcoxy en fonction hydroxyle,
h) une réaction d'oxydation de groupement comprenant un atome de soufre en fonction sulfoxyde ou sulfone correspondante,
i) une réaction de transformation de fonction sulfoxyde ou sulfone en fonction sulfoximine correspondante,
j) une réaction de transformation de radical nitrile en tétrazole,
k) une réaction de dédoublement des formes racémiques en produits dédoublés,
l) une réaction de transformation du radical carboxy en radical carbamoyle,
m) une réaction de transformation du radical carbamoyle en radical nitrile.

2. Procédé de préparation de produits de formule (I) tel que défini à la revendication 1 **caractérisé en ce que** la réaction d'un produit de formule (II) tel que défini à la revendication 1 avec un produit de formule (III) tel que défini à la revendication 1 est réalisé dans un solvant tel que le diméthylformamide, en présence d'une base de préférence, le carbonate de sodium, de potassium ou de césium à température ambiante.

3. Procédé selon la revendication 1 pour la préparation de produits de formule (I) répondant à la formule (I_{d}) : 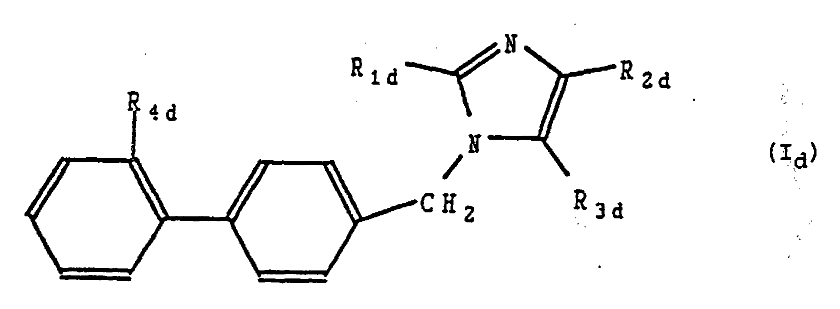 dans laquelle :
R_{1d} représente un radical alkyle renfermant au plus 4 atomes de carbone,
R_{3d} représente un radical :
carboxy libre, salifié ou estérifié par un radical alkyle linéaire ou ramifié renfermant au plus 4 atomes de carbone, formyle, acyloxy ou alkyle renfermant au plus 4 atomes de carbone éventuellement substitués par un radical hydroxyle,
R_{2d} représente un radical :
phénylthio, phénylsulfonyle, phénylsulfinyle,
alkylthio, alkylsulfonyle ou alkylsulfinyle,
dans lesquels le radical alkyle renferme au plus 4 atomes de carbone,
et R_{4d} représente le radical -SO₂-NH₂, -SO₂-NH-CO-O-R_{10d}, -SO₂-N=CH-NR_{13d}, ou -SO₂-NH-CO-NH-R_{10d} dans lesquels R_{10d} et R_{13d} identiques ou différents, sont choisis parmi l'atome d'hydrogène, le radical méthyle, éthyle, n-propyle et propényle, lesdits produits de formule (I_{d}) étant sous toutes les formes isomères possibles racémiques, énantiomères et diastéréoisomères, ainsi que les sels d'addition avec les acides minéraux et organiques ou avec les bases minérales et organiques, desdits produits de formule (I_{d}), **caractérisé en ce que** l'on utilise pour leur préparation telle que définie à la revendication 1, des produits de formules (II), (III) et (IV)
dans lesquels R'₁, R'₂, R'₃ et R'₄ ont les valeurs indiquées ci-dessus respectivement pour R_{1d'} R_{2d}, R_{3d} et R_{4d} dans lesquelles les fonctions réactives sont éventuellement protégées.

4. Procédé selon la revendication 1, **caractérisé en ce que** l'on utilise au départ un produit de formule (II) dans laquelle R'₁ représente un radical alkyle renfermant au plus 4 atomes de carbone, R'₃ représente un radical : carboxy libre, salifié ou estérifié par un radical alkyle linéaire ou ramifié renfermant au plus 4 atomes de carbone, formyle, acyloxy ou alkyle renfermant au plus 4 atomes de carbone éventuellement substitués par un radical hydroxyle,
R'₂ représente un radical :
phénylthio, phénylsulfonyle, phénylsulfinyle,
alkylthio, alkylsulfonyle ou alkylsulfinyle,
dans lesquels le radical alkyle renferme au plus 4 atomes de carbone, et les fonctions réactives sont éventuellement protégées et un produit de formule (V) dans laquelle R'₄ représente le radical -SO₂-NH₂, -SO₂-NH-CO-O-R_{10d},
-SO₂-N=CH-NR_{13d}, ou -SO₂-NH-CO-NH-R_{10d} dans lesquels R_{10d} et
R_{13d} identiques ou différents, sont choisis parmi l'atome d'hydrogène, le radical méthyle, éthyle, n-propyle et propényle, dans lesquels les fonctions réactives sont éventuellement protégées.

5. Procédé selon la revendication 1, **caractérisé en ce que** l'on utilise au départ un produit de formule (II) dans laquelle R'₃ représente un radical alcoxy ou carboxy libre, salifié ou estérifié,
et R'₂ représente un radical alkylthio ou phénylthio éventuellement oxydés sous forme de sulfoxyde ou de sulfone, ces radicaux alcoxy, alkylthio et phénylthio étant éventuellement substitués par un ou plusieurs radicaux choisis parmi les atomes d'halogène, les radicaux alkyl ou alcoxy renfermant au plus 4 atomes de carbone, trifluorométhyle, amino, mono ou dialkylamino, cyano, acyle, acyloxy ou phényl.

6. Procédé tel que défini à la revendication 1, de préparation des produits de formule (I) répondant aux formules suivantes :
- l'acide 2-butyl 1-[[2'-carboxy (1,1'-biphényl)-4-yl] méthyl] 4-(phénylthio) 1H-imidazole-5-carboxylique
- l'acide 2-butyl 1-[[2'-carboxy (1,1'-biphényl)-4-yl] méthyl] 4-(méthylthio) 1H-imidazole-5-carboxylique
- l'acide 4'-[[2-butyl 4-(éthylthio) 5-(hydroxyméthyl) 1H-imidazol-1-yl] méthyl] (1,1'-biphényl)-2-carboxylique
- l'acide 2-butyl 1-[[2'-carboxy (1,1'-biphényl)-4-yl] méthyl] 4-(éthylsulfonyl) 1H-imidazole-5-carboxylique
- l'acide 2-butyl 1-[[2'-carboxy (1,1'-biphényl)-4-yl] méthyl] 4-(éthylsulfinyl) 1H-imidazole-5-carboxylique
- l'acide 2-butyl 1-[[2'-carboxy (1,1'-biphényl)-4-yl] méthyl] 4-(éthylthio) 1H-imidazole-5-carboxylique
- l'acide 2-butyl 1-[[2'-carboxy (1,1'-biphényl)-4-yl] méthyl] 4-(phénylsulfonyl) 1H-imidazole-5-carboxylique
- l'acide 2-butyl 1-[[2'-carboxy (1,1'-biphényl)-4-yl] méthyl] 4-(phénylsulfinyl) 1H-imidazole-5-carboxylique
- l'acide 2-butyl 1-[[2'-tétrazolyl (1,1'-biphényl)-4-yl] méthyl] 4-(méthylthio) lH-imidazole-5-carboxylique,
- le 2-butyl 4-(méthylthio) 1-[[2'-((((propylamino) carbonyl) amino) sulfonyl) (1,1'-biphényl)-4-yl] méthyl]-1H-imidazole 5-carboxylate d'éthyle,
- l'acide 2-butyl 4-(méthylthio) 1-[(2'-((((propylamino) carbonyl) amino) sulfonyl) (1,1'-biphényl)-4-yl] méthyl]-1H-imidazole 5-carboxylique,
- l'acide 2-butyl 4-(méthylthio) 1-[[2'-((((propylamino) carbonyl) amino) sulfonyl) (1,1'-biphényl)-4-yl] méthyl]-1H-imidazole 5-carboxylique, di sel de potassium.

7. Procédé tel que défini à la revendication 1, de préparation des produits de formule (I) répondant aux formules suivantes :
- le 2-butyl 4-(méthylthio) 1-[[2'-((((propylamino) carbonyl) amino) sulfonyl) (1,1'-biphényl)-4-yl] méthyl]-1H-imidazole 5-carboxylate d'éthyle,
- l'acide 2-butyl 4-(méthylthio) 1-[[2'-((((propylamino) carbonyl) amino) sulfonyl) (1,1'-biphényl)-4-yl] méthyl]-1H-imidazole 5-carboxylique,
- l'acide 2-butyl 4-(méthylthio) 1-[[2'-((((propylamino) carbonyl) amino) sulfonyl) (1,1'-biphényl)-4-yl] méthyl]-1H-imidazole 5-carboxylique, di sel de potassium.

8. A titre de produits industriels nouveaux, les composés de formule (IV) : 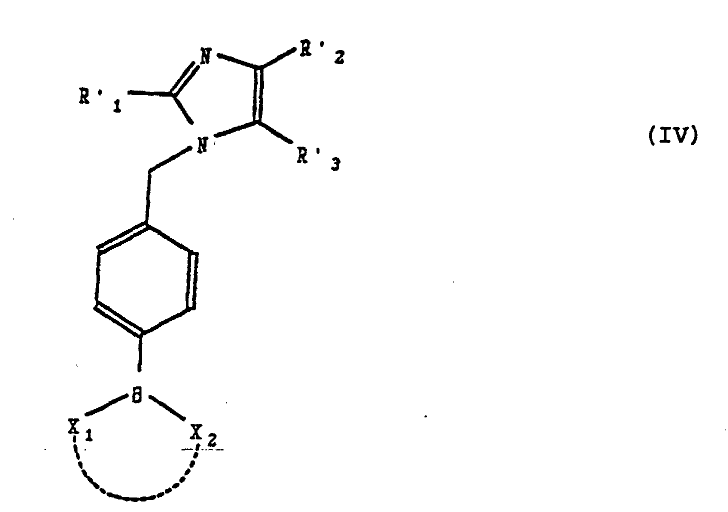 dans laquelle R'₁, R'₂, R'₃, X₁, X₂ et B ont les significations indiquées à la revendication 1.

9. A titre de produits industriels nouveaux, les produits :
**4b -** 2-[4-(bromométhyl) phényl] 1,3,2-dioxaborolane
**5 -** 1-[(4-boronophényl) méthyl] 2-butyl 4-(méthylthio) 1H-imidazole 5-carboxylate d'éthyle
**6a -** 2-butyl 1-[4-(5,5-diméthyl 1,3,2-dioxaborolan-2-yl) phénylméthyl] 4-(méthylthio) 1H-imidazole 5-carboxylate d'éthyle
**6b -** 2-butyl 1-[4-(1,3,2-dioxaborolan 2-yl) phényl méthyl] 4-(méthylthio) 1H-imidazole 5-carboxylate d'éthyle
**7 -** (T-4)((4-((2-butyl 4-(méthylthio) 5-(éthoxycarbonyl) 1H-imidazole 1-yl((méthyl) phényl) ((2,2'-(méthylimino) bis-(éthanolato)) (2-)-N,O,O')-bore
**8** - 2-butyl 1-((4-(1,3,2-benzodioxaborol-2-yl) phényl) méthyl) 4-(méthylthio) 1H-imidazole 5-carboxylate d'éthyle
**9a** - 2-butyl 1-((4-(4,4,5,5-tétraméthyl 1,3,2-dioxolaborolan-2-yl) phényl) méthyl) 4-(méthylthio) 1H-imidazole 5-carboxylate d'éthyle
**9b** - 2-butyl 1-((4-(1,3,2-dioxolaborolan-2-yl) phényl) méthyl) 4-(méthylthio) 1H-imidazole 5-carboxylate d'éthyle.

## Claims

1. Process for the preparation of products of formula (I): 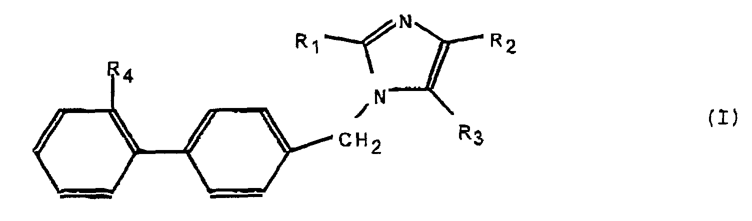 in which:
R₁ represents an alkyl radical containing at most 4 carbon atoms,
R₃ represents a hydrogen atom, a formyl radical an optionally substituted, acyloxy, alkyl or alkoxy radical, or a carboxy radical, free, salified or esterified by an alkyl radical,
R₂ represents a phenylthio, phenylsulphonyl, phenylsulphinyl, alkylthio, alkylsulphonyl or alkylsulphinyl radical, optionally substituted, such that in all these radicals which can be represented by R₂ and R₃, the alkyl, alkoxy radicals containing at most 6 carbon atoms, and the phenyl radicals are optionally substituted by one or more radicals chosen from the halogen atoms and the hydroxyl, trifluoromethyl, acyloxy, carboxy radicals free, salified or esterified by an alkyl, phenyl, pyridyl, tetrazolyl, alkyl and alkoxy radical containing at most 4 carbon atoms and themselves optionally substituted by an alkoxy radical containing at most 4 carbon atoms,
R₄ represents the cyano, carboxy radical, free, salified or esterified by an alkyl radical, the -SO₂-X-R₁₀ radical in which X represents the -NH-, -NH-CO-, -NH-CO-O-, -N=CH-N-R13 -NH-CO-NH- radicals or a single bond and R₁₀ and R₁₃ identical or different, represent a hydrogen atom, a methyl, ethyl, propyl, vinyl, allyl, pyridyl, phenyl, benzyl, nitropyridyl, pyrimidyl, tetrazolyl, diazolyl, piperidinyl, alkylpiperidinyl, thiazolyl, alkylthiazolyl, tetrahydrofuranyl, methyltetrahydrofuranyl radical, said products of formula (I) being in all racemic, enantiomeric and diastereoisomeric isomer forms possible, as well as the addition salts with mineral and organic acids or with mineral and organic bases of said products of formula (I), **characterized in that** a compound of formula (II): 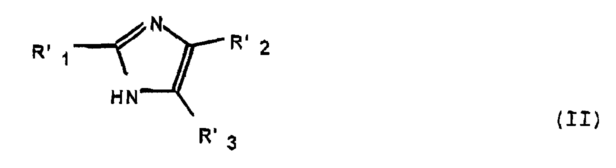
in which R'₁, R'₂ and R'₃ have the meanings indicated above respectively for R₁, R₂ and R₃ and in which the optional reactive functions are optionally protected, is reacted with a compound of formula (III): 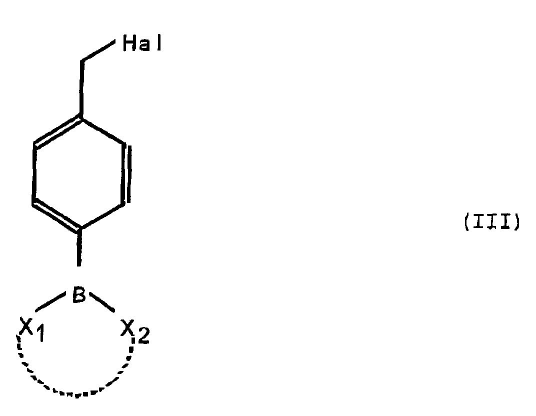 in which Hal represents a halogen atom, B represents a boron atom and X₁, X₂ are such that:
either X₁ and X₂ identical or different represent a hydroxyl, alkyl or alkoxy radical containing at most 6 carbon atoms, a phenyl or phenoxy radical,
or X₁ with X₂ form with the boron atom to which they are linked a ring chosen from 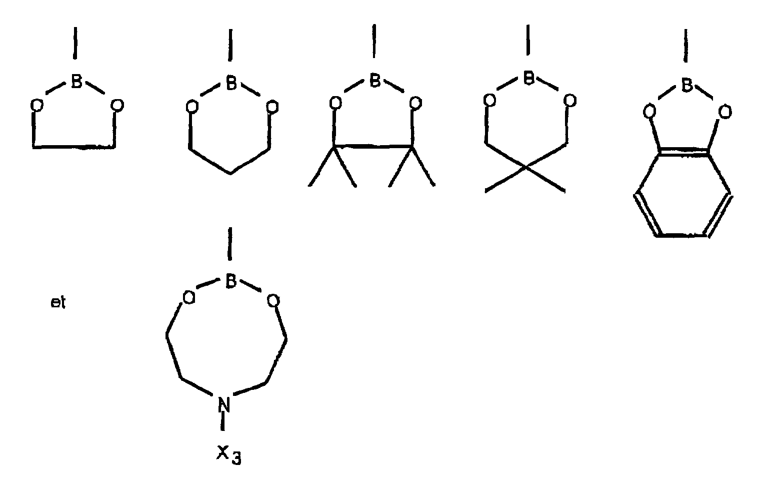
X₃ representing a hydrogen atom or an alkyl radical containing at most 4 carbon atoms, in order to obtain a product of formula (IV): 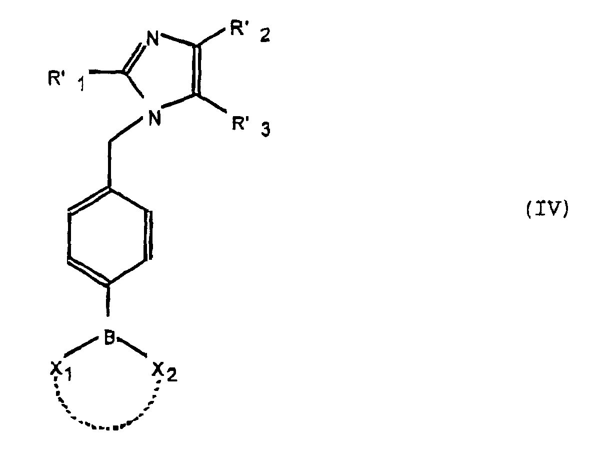
in which R'₁, R'₂, R'₃, X₁, X₂ and B have the meanings indicated above, which is reacted with a product of formula (V): 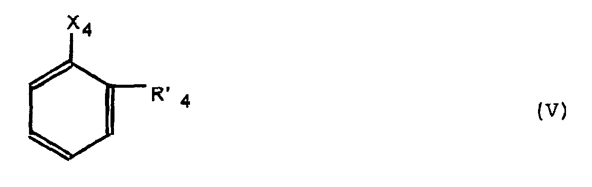 in which X₄ represents a halogen atom, an alkoxy, triflate or -O-SO₂F radical and R'₄ has the meaning indicated above for R₄ in which the optional reactive functions are optionally protected, in order to obtain a product of formula (I'): 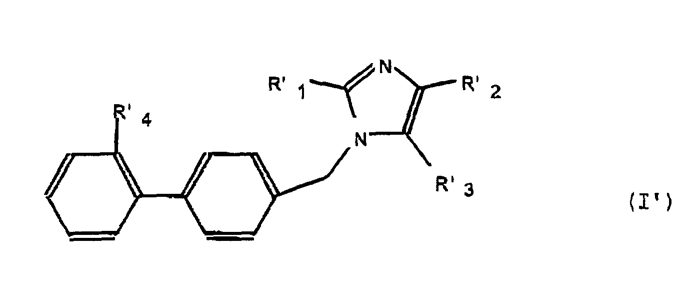 product of formula (I') which is subjected, if desired and if necessary, to one or more of the following reactions, in any order:
a) an elimination reaction of the protective groups that can be carried by the protected reactive functions,
b) a salification reaction by a mineral or organic acid or by a base,
c) an esterification reaction of the acid function,
d) a saponification reaction of the ester function,
e) a conversion reaction of the cyano function to an acid function,
f) a reduction reaction of the carboxy function to an alcohol function,
g) a conversion reaction of the alkoxy function to a hydroxyl function,
h) an oxidation reaction of groups comprising a sulphur atom to a corresponding sulphoxide or sulphone function,
i) a conversion reaction of the sulphoxide or sulphone function to a corresponding sulphoximine function,
j) a conversion reaction of a nitrile radical to a tetrazol radical,
k) a resolution reaction of the racemic forms to resolved forms,
l) a conversion reaction of the carboxy radical to a carbamoyl radical,
m) a conversion reaction of the carbamoyl radical to a nitrile radical.

2. Process for the preparation of products of formula (I) as defined in claim 1 **characterized in that** the reaction of a product of formula (II) as defined in claim 1 with a product of formula (III) as defined in claim 1 is carried out in a solvent such as dimethylformamide in the presence of a base preferably sodium, potassium or cesium carbonate at ambient temperature.

3. Process according to claim 1 for the preparation of products of formula (I) corresponding to formula (I_{d}): 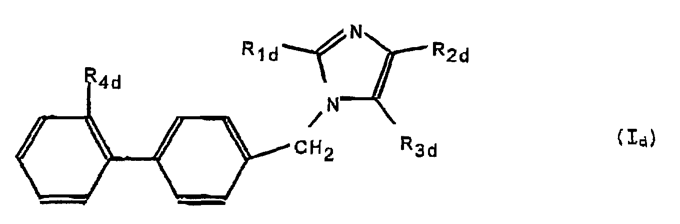 in which:
R_{1d} represents an alkyl radical containing at most 4 carbon atoms,
R_{3d} represents a:
carboxy radical, free, salified or esterified by a linear or branched alkyl containing at most 4 carbon atoms, formyl, acyloxy or alkyl radical containing at most 4 carbon atoms optionally substituted by a hydroxyl radical,
R_{2d} represents a:
phenylthio, phenylsulphonyl, phenylsulphinyl, alkylthio, alkylsulphonyl or alkylsulphinyl radical,
in which the alkyl radical contains at most 4 carbon atoms,
and R_{4d} represents the -SO₂-NH₂, -SO₂-NH-CO-O-R_{10d}, -SO₂-N=CH-NR_{13d}, or -SO₂-NH-CO-NH-R_{10d} radical in which R_{10d} and R_{13d} identical or different, are chosen from the hydrogen atom, the methyl, ethyl, n-propyl and propenyl radical, the said products of formula (I_{d}) being in all possible racemic, enantiomeric and diastereoisomeric isomer forms, as well as the addition salts with mineral and organic acids or with mineral and organic bases of the said products of formula (I_{d}), **characterized in that** products of formulae (II), (III) and (IV) in which R'₁, R'₂, R'₃ and R'₄ have the values indicated above respectively for R_{1d}, R_{2d}, R_{3d} and R_{4d} in which the reactive functions are optionally protected are used for their preparation as defined in claim 1.

4. Process according to claim 1, **characterized in that** at the start a product of formula (II) is used in which R'₁ represents an alkyl radical containing at most 4 carbon atoms, R'₃ represents a carboxy radical, free, salified or esterified by a linear or branched alkyl radical containing at most 4 carbon atoms, formyl, acyloxy or alkyl radical containing at most 4 carbon atoms optionally substituted by a hydroxyl radical,
R'₂ represents a:
phenylthio, phenylsulphonyl, phenylsulphinyl, alkylthio, alkylsulphonyl or alkylsulphinyl radical,
in which the alkyl radical contains at most 4 carbon atoms, and the reactive functions are optionally protected and a product of formula (V) in which R'₄ represents the -SO₂-NH₂,-SO₂-NH-CO-O-R_{10d}, -SO₂-N=CH-NR_{13d}, or -SO₂-NH-CO-NH-R_{10d} radical
in which R_{10d} and
R_{13d} identical or different, are chosen from the hydrogen atom, the methyl, ethyl, n-propyl and propenyl radical, in which the reactive functions are optionally protected.

5. Process according to claim 1, **characterized in that** at the start a product of formula (II) is used in which R'₃ represents an alkoxy or a free, salified or esterified carboxy radical,
and R'₂ represents an alkylthio or phenylthio radical optionally oxidized in the form of sulphoxide or sulphone, these alkoxy, alkylthio and phenylthio radicals being optionally substituted by one or more radicals chosen from the halogen atoms, alkyl or alkoxy containing at most 4 carbon atoms, trifluoromethyl, amino, mono or dialkylamino, cyano, acyl, acyloxy or phenyl radicals.

6. Process as defined in claim 1, for the preparation of the products of formula (I) corresponding to the following formulae:
- 2-butyl 1-[[2'-carboxy (1,1'-biphenyl)-4-yl] methyl] 4-(phenylthio) 1H-imidazole-5-carboxylic acid
- 2-butyl 1-[[2'-carboxy (1,1'-biphenyl)-4-yl] methyl] 4-(methylthio) 1H-imidazole-5-carboxylic acid
- 4'-[[2-butyl 4-(ethylthio) 5-(hydroxymethyl) 1H-imidazole-1-yl] methyl] (1,1'-biphenyl)-2-carboxylic acid
- 2-butyl 1-[[2'-carboxy (1,1'-biphenyl)-4-yl] methyl] 4-(ethylsulphonyl) 1H-imidazole-5-carboxylic acid
- 2-butyl 1-[[2'-carboxy (1,1'-biphenyl)-4-yl] methyl] 4-(ethylsulphinyl) 1H-imidazole-5-carboxylic acid
- 2-butyl 1-[[2'-carboxy (1,1'-biphenyl)-4-yl] methyl] 4-(ethylthio) 1H-imidazole-5-carboxylic acid
- 2-butyl 1-[[2'-carboxy (1,1'-biphenyl)-4-yl] methyl] 4-(phenylsulphonyl) 1H-imidazole-5-carboxylic acid
- 2-butyl 1-[[2'-carboxy (1,1'-biphenyl)-4-yl] methyl] 4-(phenylsulphinyl) 1H-imidazole-5-carboxylic acid
- 2-butyl 1-[[2'-tetrazolyl (1,1'-biphenyl)-4-yl] methyl] 4-(methylthio) 1H-imidazole-5-carboxylic acid,
- ethyl 2-butyl 4-(methylthio) 1-[[2'-((((propylamino) carbonyl) amino) sulphonyl) (1,1'-biphenyl)-4-yl] methyl]-1H-imidazole 5-carboxylate,
- 2-butyl 4-(methylthio) 1-[[2'-((((propylamino) carbonyl) amino) sulphonyl) (1,1'-biphenyl)-4-yl] methyl]-1H-imidazole 5-carboxylic acid,
- 2-butyl 4-(methylthio) 1-[[2'-((((propylamino) carbonyl) amino) sulphonyl) (1,1'-biphenyl)-4-yl] methyl]-1H-imidazole 5-carboxylic acid di-potassium salt.

7. Process as defined in claim 1, for the preparation of products of formula (I) corresponding to the following formulae:
- ethyl 2-butyl 4-(methylthio) 1-[[2'-((((propylamino) carbonyl) amino) sulphonyl) (1,1'-biphenyl)-4-yl] methyl]-1H-imidazole-5-carboxylate,
- 2-butyl 4-(methylthio) 1-[[2'-((((propylamino) carbonyl) amino) sulphonyl) (1,1'-biphenyl)-4-yl] methyl]-1H-imidazole 5-carboxylic acid,
- 2-butyl 4-(methylthio) 1-[[2'-((((propylamino) carbonyl) amino) sulphonyl) (1,1'-biphenyl)-4-yl] methyl]-1H-imidazole 5-carboxylic acid, di-potassium salt.

8. As new industrial products, the compounds of formula (IV) : 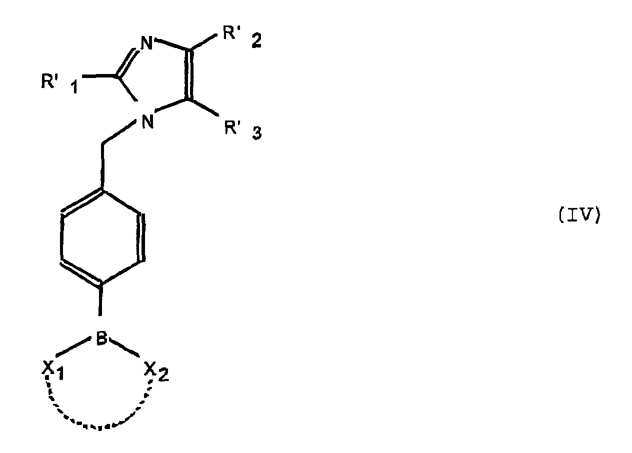 in which R'₁, R'₂, X₁, X₂ and B have the meanings indicated in claim 1.

9. As new industrial products, the products:
**4b** - ethyl 2-[4-(bromomethyl) phenyl] 1,3,2-dioxaborolane
**5** - ethyl 1-[4-(boronophenyl) methyl] 2-butyl 4-(methylthio) 1H-imidazole 5-carboxylate
**6a** - ethyl 2-butyl 1-[4-(5,5,dimethyl 1,3,2-dioxaborolan-2-yl) phenylmethyl] 4-(methylthio) 1H-imidazole 5-carboxylate
**6b** - ethyl 2-butyl 1-[4-(1,3,2-dioxaborolan 2-yl) phenyl methyl] 4-(methylthio) 1H-imidazole 5-carboxylate
**7** - (T-4)((4-((2-butyl 4-(methylthio) 5-(ethoxycarbonyl) 1H-imidazole 1-yl((methyl) phenyl) ((2,2'-(methylimino) bis(ethanolato)) (2-)-N,O,O')-boron
**8** - ethyl 2-butyl 1-((4-(1,3,2-benzodioxaborol-2-yl) phenyl) methyl) 4-(methylthio) 1H-imidazole 5-carboxylate
**9a** - ethyl 2-butyl 1-((4-(4,4,5,5-tetramethyl 1,3,2-dioxolaborolan-2-yl) phenyl) methyl) 4-(methylthio) 1H-imidazole 5-carboxylate
**9b** - ethyl 2-butyl 1-((4-(1,3,2-dioxolaborolan-2-yl) phenyl) methyl) 4-(methylthio) 1H-imidazole 5-carboxylate.

## Patentansprüche

1. Verfahren zur Herstellung von Produkten der Formel (I) 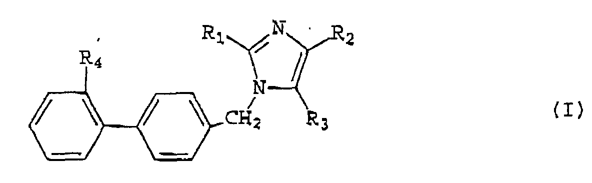 in der
R₁ einen Rest Alkyl mit höchstens 4 Kohlenstoffatomen darstellt, R₃ ein Wasserstoffatom, einen Rest Formyl, Acyloxy, Alkyl oder Alkoxy, gegebenenfalls substituiert, oder einen Rest Carboxy, frei, in Salz überführt oder verestert durch einen Rest Alkyl, bedeutet,
R₂ einen Rest Phenylthio, Phenylsulfonyl, Phenylsulfinyl, Alkylthio, Alkylsulfonyl oder Alkylsulfinyl darstellt, gegebenenfalls substituiert, wie in allen diesen Resten, die von R₂ und R₃ dargestellt werden können, sind die Reste Alkyl, Alkoxy mit höchstens 6 Kohlenstoffatomen, und die Reste Phenyl gegebenenfalls substituiert durch einen oder mehrere Reste, ausgewählt unter den Halogenatomen und den Resten Hydroxyl, Trifluormethyl, Acyloxy, Carboxy, frei, in Salz überführt oder verestert durch einen Rest Alkyl, Phenyl, Pyridyl, Tetrazolyl, Alkyl und Alkoxy mit höchstens 4 Kohlenstoffatomen, gegebenenfalls selbst substituiert durch einen Rest Alkoxy mit höchstens 4 Kohlenstoffatomen,
R₄ den Rest Cyano, Carboxy, frei, in Salz überführt oder verestert durch einen Rest Alkyl, den Rest -SO₂-X-R₁₀ darstellt, worin X die Reste -NH-, -NH-CO-, -NH-CO-, -N=CH-N-R₁₃, -NH-CO-NH- oder eine einfache Bindung bedeutet, und R₁₀ und R₁₃, gleich oder verschieden, ein Wasserstoffatom, einen Rest Methyl, Ethyl, Propyl, Vinyl, Allyl, Pyridyl, Phenyl, Benzyl, Nitropyridyl, Pyrimidyl, Tetrazolyl, Diazolyl, Piperidinyl, Alkylpiperidinyl, Thiazolyl, Alkylthiazolyl, Tetrahydrofuranyl, Methyltetrahydrofuranyl darstellen,
wobei die genannten Produkte der Formel (I) in allen möglichen isomeren racemischen Formen, Enantiomeren und Diastereoisomeren vorliegen können, sowie die Additionssalze der genannten Produkte der Formel (I) mit Mineralsäuren und organischen Säuren oder mit Mineralbasen und organischen Basen, **dadurch gekennzeichnet, daß** man eine Verbindung der Formel (II) 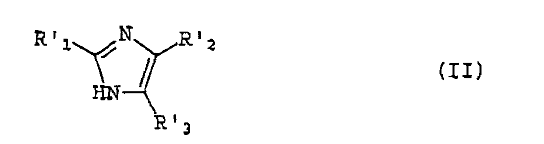 in der R'₁, R'₂ und R'₃ die oben jeweils für R₁, R₂ und R₃ angegebenen Bedeutungen besitzen und in denen die eventuellen reaktiven Funktionen gegebenenfalls geschützt sind, mit einer Verbindung der Formel (III) 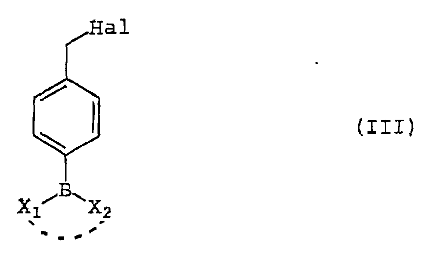 zur Reaktion bringt, in der Hal ein Halogenatom ist, B ein Boratom darstellt und X₁, X₂ derart sind, daß
entweder X₁ und X₂, gleich oder verschieden, einen Rest Hydroxyl, Alkyl oder Alkoxy mit höchstens 6 Kohlenstoffatomen, einen Rest Phenyl oder Phenoxy bedeuten, oder
X₁ und X₂ zusammen mit dem Boratom, an das sie gebunden sind, einen Ring bilden, ausgewählt unter 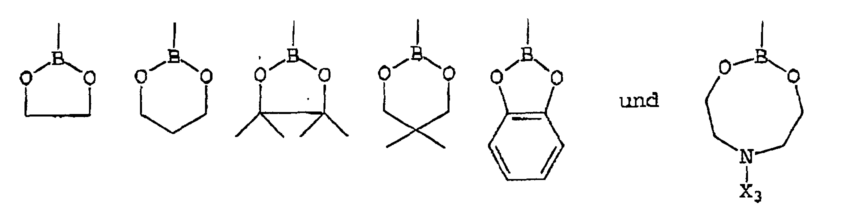 worin X₃ ein Wasserstoffatom oder einen Rest Alkyl mit höchstens 4 Kohlenstoffatomen darstellt, um ein Produkt der Formel (IV) 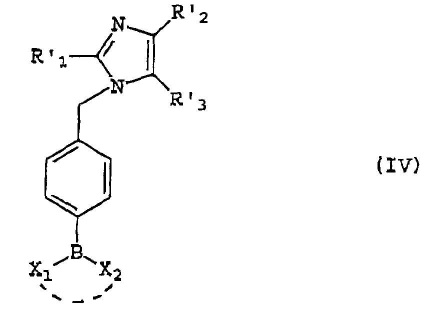 zu erhalten, in der R'₁, R'₂, R'₃, X₁, X₂ und B die oben angegebenen Bedeutungen besitzen, das man mit einem Produkt der Formel (V) 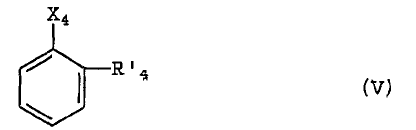 zur Reaktion bringt, in der X₄ ein Halogenatom, einen Rest Alkoxy, Triflat oder -O-SO₂F darstellt und R'₄ die oben bei R₄ angegebene Bedeutung besitzt, worin die eventuellen reaktiven Funktionen gegebenenfalls geschützt sind, um ein Produkt der Formel (I') 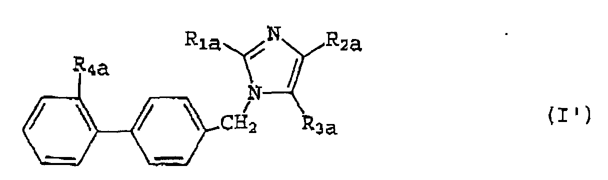 zu erhalten, das man, wenn erwünscht und wenn erforderlich, einer oder mehreren der folgenden Reaktionen in irgendeiner Reihenfolge unterzieht:
a) einer Reaktion zur Entfernung der Schutzgruppen, die von den geschützten reaktiven Funktionen getragen werden können,
b) einer Reaktion zur Salzbildung durch eine Mineralsäure oder organische Säure oder durch eine Base,
c) einer Reaktion zur Veresterung der Säurefunktion,
d) einer Reaktion zur Verseifung der Esterfunktion,
e) einer Reaktion zur Umwandlung der Cyanofunktion zur Säurefunktion,
f) einer Reaktion zur Reduktion der Carboxyfunktion zur Alkoholfunktion,
g) einer Reaktion zur Umwandlung der Alkoxyfunktion zur Hydroxylfunktion,
h) einer Reaktion zur Oxidation der Gruppe, die ein Schwefelatom umfaßt, zur entsprechenden Sulfoxidfunktion oder Sulfonfunktion,
i) einer Reaktion zur Umwandlung der Sulfoxidfunktion oder der Sulfonfunktion zur entsprechenden Sulfoximinfunktion,
j) einer Reaktion zur Umwandlung des Restes Nitril zum Tetrazol,
k) einer Reaktion zur Aufspaltung der racemischen Formen zu den Spaltprodukten,
l) einer Reaktion zur Umwandlung des Restes Carboxy zum Rest Carbamoyl,
m) einer Reaktion zur Umwandlung des Restes Carbamoyl zum Rest Nitril.

2. Verfahren zur Herstellung der Produkte der Formel (I) wie in Anspruch 1 definiert, **dadurch gekennzeichnet, daß** die Reaktion eines wie in Anspruch 1 definierten Produktes der Formel (II) mit einem wie in Anspruch 1 definierten Produkt der Formel (III) in einem Lösungsmittel wie Dimethylformamid, in Anwesenheit einer Base, vorzugsweise Natriumcarbonat, Kaliumcarbonat oder Cäsiumcarbonat, und bei Umgebungstemperatur realisiert wird.

3. Verfahren nach Anspruch 1 zur Herstellung der Produkte der Formel (I), die der Formel (I_{d}) 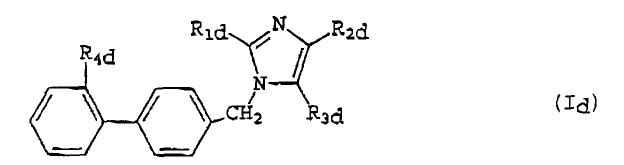 entsprechen, in der
R_{1d} einen Rest Alkyl mit höchstens 4 Kohlenstoffatomen darstellt,
R_{3d} einen Rest Carboxy, frei, in Salz überführt oder verestert durch einen linearen oder verzweigten Rest Alkyl mit höchstens 4 Kohlenstoffatomen, Formyl, Acyloxy oder Alkyl mit höchstens 4 Kohlenstoffatomen bedeutet, gegebenenfalls substituiert durch einen Rest Hydroxyl,
R_{2d} einen Rest Phenylthio, Phenylsulfonyl, Phenylsulfinyl, Alkylthio, Alkylsulfonyl oder Alkylsulfinyl darstellt, worin der Rest Alkyl höchstens 4 Kohlenstoffatome umfaßt, und
R_{4d} den Rest -SO₂-NH₂, SO₂-NH-CO-O-R_{10d}, -SO₂-N=CH-NR_{13d} oder -SO₂-NH-CO-NH-R_{10d} darstellt, worin R_{10d} und R_{13d}, gleich oder verschieden, ausgewählt werden unter dem Wasserstoffatom, dem Rest Methyl, Ethyl, n-Propyl und Propenyl, wobei die genannten Produkte der Formel (I_{d}) in allen möglichen isomeren racemischen Formen, Enantiomeren und Diastereoisomeren vorliegen können, sowie die Additionssalze der genannten Produkte der Formel (I_{d}) mit Mineralsäuren und organischen Säuren oder mit Mineralbasen und organischen Basen, **dadurch gekennzeichnet, daß** man für ihre wie in Anspruch 1 definierte Herstellung Produkte der Formeln (II), (III) und (IV) verwendet, in denen R'₁, R'₂, R'₃ und R'₄ die oben jeweils für R_{1d}, R_{2d}, R_{3d} und R_{4d} angegebenen Werte besitzen, worin die reaktiven Funktionen gegebenenfalls geschützt sind.

4. Verfahren nach Anspruch 1, **dadurch gekennzeichnet, daß** man als Ausgangsprodukt ein Produkt der Formel (II), in der R'₁ einen Rest Alkyl mit höchstens 4 Kohlenstoffatomen darstellt,
R'₃ einen Rest Carboxy, frei, in Salz überführt oder verestert durch einen linearen oder verzweigten Rest Alkyl mit höchstens 4 Kohlenstoffatomen, Formyl, Acyloxy oder Alkyl mit höchstens 4 Kohlenstoffatomen bedeutet, gegebenenfalls substituiert durch einen Rest Hydroxyl,
R'₂ einen Rest Phenylthio, Phenylsulfonyl, Phenylsulfinyl, Alkylthio, Alkylsulfonyl oder Alkylsulfinyl darstellt, worin der Rest Alkyl höchstens 4 Kohlenstoffatome umfaßt, und die reaktiven Funktionen gegebenenfalls geschützt sind,
und ein Produkt der Formel (V) verwendet, in der R'₄ den Rest -SO₂-NH₂, -SO₂-NH-CO-O-R_{10d}, -SO₂-N=CH-NR_{13d} oder -SO₂-NH-CO-NH-R_{10d} darstellt, worin R_{10d} und R_{13d}, gleich oder verschieden, ausgewählt werden unter dem Wasserstoffatom, dem Rest Methyl, Ethyl, n-Propyl und Propenyl, worin die reaktiven Funktionen gegebenenfalls geschützt sind.

5. Verfahren nach Anspruch 1, **dadurch gekennzeichnet, daß** man als Ausgangsprodukt ein Produkt der Formel (II) verwendet, in der R'₃ einen Rest Alkoxy oder Carboxy, frei, in Salz überführt oder verestert darstellt und
R'₂ einen Rest Alkylthio oder Phenylthio bedeutet, gegebenenfalls oxidiert in Form von Sulfoxid oder Sulfon, wobei diese Reste Alkoxy, Alkylthio und Phenylthio gegebenenfalls substituiert sind durch ein oder mehrere Reste, ausgewählt unter den Halogenatomen, den Resten Alkyl und Alkoxy mit höchstens 4 Kohlenstoffatomen, Trifluormethyl, Amino, Mono- oder Dialkylamino, Cyano, Acyl, Acyloxy oder Phenyl.

6. Verfahren wie in Anspruch 1 definiert zur Herstellung der Produkte der Formel (I), die den folgenden Formeln entsprechen:
- 2-Butyl-1-{[2'-carboxy-(1,1'-biphenyl)-4-yl]-methyl}-4-(phenylthio)-1H-imidazol-5-carbonsäure,
- 2-Butyl-1-{[2'-carboxy-(1,1'-biphenyl)-4-yl]-methyl}-4-(methylthio)-1H-imidazol-5-carbonsäure,
- 4'-{[2-Butyl-4-(ethylthio)-5-(hydroxymethyl)-1H-imidazol-1-yl]-methyl}-(1,1'-biphenyl)-2-carbonsäure,
- 2-Butyl-1-{[2'-carboxy-(1,1'-biphenyl)-4-yl]-methyl}-4-(ethylsulfonyl)-1H-imidazol-5-carbonsäure,
- 2-Butyl-1-{[2'-carboxy-(1,1'-biphenyl)-4-yl]-methyl}-4-(ethylsulfinyl)-1H-imidazol-5-carbonsäure,
- 2-Butyl-1-{[2'-carboxy-(1,1'-biphenyl)-4-yl]-methyl}-4-(ethylthio)-1H-imidazol-5-carbonsäure,
- 2-Butyl-1-{[2'-carboxy-(1,1'-biphenyl)-4-yl]-methyl}-4-(phenylsulfonyl)-1H-imidazol-5-carbonsäure,
- 2-Butyl-1-{[2'-carboxy-(1,1'-biphenyl)-4-yl]-methyl}-4-(phenylsulfinyl)-1H-imidazol-5-carbonsäure,
- 2-Butyl-1-{[2'-tetrazolyl-(1,1'-biphenyl)-4-yl]-methyl}-4-(methylthio)-1H-imidazol-5-carbonsäure,
- 2-Butyl-4-(methylthio)-1-{{2'-[[((propylamino)-carbonyl)-amino]-sulfonyl]-(1,1'-biphenyl)-4-yl}-methyl}-1H-imidazol-5-carbonsäure-ethylester,
- 2-Butyl-4-(methylthio)-1-{{2'-[[((propylamino)-carbonyl)-amino]-sulfonyl]-(1,1'-biphenyl)-4-yl}-methyl}-1H-imidazol-5-carbonsäure,
- 2-Butyl-4-(methylthio)-1-{{2'-[[((propylamino)-carbonyl)-amino]-sulfonyl]-(1,1'-biphenyl)-4-yl}-methyl}-1H-imidazol-5-carbonsäure-Dikaliumsalz.

7. Verfahren wie in Anspruch 1 definiert zur Herstellung der Produkte der Formel (I), die den folgenden Formeln entsprechen:
- 2-Butyl-4-(methylthio)-1-{{2'-[[((propylamino)-carbonyl)-amino]-sulfonyl]-(1,1'-biphenyl)-4-yl}-methyl}-1H-imidazol-5-carbonsäure-ethylester,
- 2-Butyl-4-(methylthio)-1-{{2'-[[((propylamino)-carbonyl)-amino]-sulfonyl]-(1,1'-biphenyl)-4-yl}-methyl}-1H-imidazol-5-carbonsäure,
- 2-Butyl-4-(methylthio)-1-{{2'-[[((propylamino)-carbonyl)-amino]-sulfonyl]-(1,1'-biphenyl)-4-yl}-methyl}-1H-imidazol-5-carbonsäure-Dikaliumsalz.

8. Als neue industrielle Produkte die Verbindungen der Formel (IV): 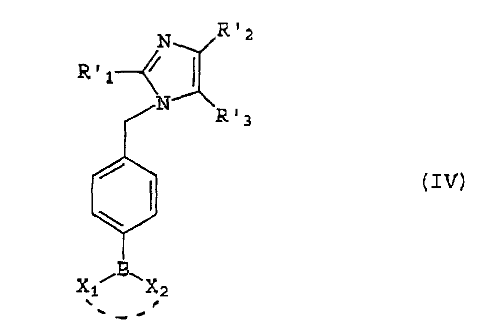 in der R'₁, R'₂, R'₃, X₁, X₂ und B die in Anspruch 1 angegebenen Bedeutungen besitzen.

9. Als neue industrielle Produkte die Produkte:
4b - 2-[4-(Brommethyl)-phenyl]-1,3,2-dioxaborolan,
5 - 1-[(4-Boronophenyl)-methyl]-2-butyl-4-(methylthio)-1H-imidazol-5-carbonsäure-ethylester,
6a - 2-Butyl-1-[4-(5,5-dimethyl-1,3,2-dioxaborolan-2-yl)-phenyl-methyl]-4-(methylthio)-1H-imidazol-5-carbonsäure-ethylester,
6b - 2-Butyl-1-[4-(1,3,2-dioxaborolan-2-yl)-phenylmethyl]-4-(methylthio)-1H-imidazol-5-carbonsäure-ethylester,
7 - (T-4) [4-[[2-Butyl-4-(methylthio)-5-(ethoxycarbonyl)-1H-imidazol-1-yl]-methyl]-phenyl}-[[2,2'-(methylimino)-bis-(ethanolato)]-(2-)-N,O,O']-bor
8 - 2-Butyl-1-{[4-(1,3,2-benzodioxaborol-2-yl)-phenyl]-methyl}-4-(methylthio)-1H-imidazol-5-carbonsäure-ethylester
9a - 2-butyl-1-{[4-(4,4,5,5-tetramethyl-1,3,2-dioxolaborolan-2-yl)-phenyl)-methyl}-4-(methylthio)-1H-imidazole-5-carbonsäure-ethylester
9b - 2-butyl-1-{[4-(1,3,2-dioxolaborolan-2-yl)-phenyl]-methyl}-4-(methylthio)-1H-imidazole-5-carbonsäure-ethylester
